# EUROPEAN PATENT SPECIFICATION

(11) **EP 1 888 120 B1**
(45) Date of publication and mention of the grant of the patent: **05.12.2012**
(21) Application number: 06756205.8
(22) Date of filing: 07.06.2006
(51) Int. Cl.: A61K 47/48, A61P 25/00, A61P 35/00, C07C 69/24, C07D 279/28

(54) **NOVEL SALTS OF CONJUGATED PSYCHOTROPIC DRUGS AND PROCESSES OF PREPARING SAME**
NEUE SALZE VON KONJUGIERTEN PSYCHOTROPEN ARZNEIMITTELN UND VERFAHREN ZU IHRER HERSTELLUNG
NOUVEAUX SELS DE MEDICAMENTS PSYCHOTROPES CONJUGUES ET LEURS PROCEDES DE PREPARATION

(30) Priority: 07.06.2005 US 687851 P; 25.04.2006 US 794501 P
(43) Date of publication of application: 20.02.2008
(60) Divisional application: 10013191.1
(73) Proprietor: Ramot at Tel Aviv University Ltd., 69975 Tel Aviv (IL); BAR-ILAN UNIVERSITY, 52900 Ramat Gan (IL); BIOLINERX LTD., 91450 Jerusalem (IL)
(72) Inventor: NUDELMAN, Abraham, 76284 Rechovot (IL); REPHAELI, Ada, 46726 Herzlia (IL); GIL-AD, Irit, 46420 Herzlia (IL); WEIZMAN, Abraham, 69341 Tel-Aviv (IL); HALACHMI, Shlomit, 30500 Binyamina (IL); BENJAMIN, Eran, J., 76468 Rechovot (IL)
(74) Representative: Fickert, Stefan Christopher
(86) International application number: PCT/IL2006/000666
(87) International publication number: WO 2006/131923

(56) References cited:
- WO-A-99/26661
- WO-A-03/026563
- WO-A-2005/092392
- GB-A- 1 514 312
- US-A- 3 956 493
- US-A- 3 978 216
- GIL-AD I ET AL: "NOVEL ANTI-PSYCHOTICS THAT DISPLAY GABAERGIC ACTIVITY AND DECREASED EXTRAPYRAMIDAL SIDE EFFECTS FOR THE TREATMENT OF SCHIZOPHRENIA AND RELATED PSYCHIATRIC DISORDERS" NEURAL PLASTICITY, FREUND PUBLISHING HOUSE, TEL AVIV, IL, vol. 10, no. 3, 2003, page 200, XP008064103 ISSN: 0792-8483
- SAKAMOTO, F. ET AL.: "Studies on prodrugs VI. Preparation and characterization of 5 substituted 2 oxo-1 3-dioxol-4-ylmethyl esters of mecillinam" CHEMICAL AND PHARMACEUTICAL BULLETIN, vol. 35, no. 2, 1987, pages 642-646, XP008078018
- WARE E. C. AND LU R.: "An automated approach to salt selection for new Unique trazodone salts" PHARMACEUTICAL RESEARCH, vol. 21, no. 1, 2004, pages 177-184, XP007902093

## Description

### FIELD AND BACKGROUND OF THE INVENTION

The present invention relates to novel chemical conjugates of psychotropic drugs and organic acids, preparation and uses thereof as defined in the annexed claim. More particularly, the present invention relates to novel acid addition salts of such chemical conjugates, which are particularly characterized by high *ex-vivo* stability, to novel processes of preparing the conjugates and the acid addition salts thereof and to uses thereof in the treatment of psychotic disorders and diseases.

Psychotropic drugs are pharmacological agents that act mainly in the central nervous system (CNS) by modulating neuronal signals transduction. Psychotropic drugs are therefore known, and are referred to herein, as pharmacological agents, which are able to cross the blood-brain barrier (BBB) and exert an activity in the CNS to thereby treat CNS associated impairments and include, for example, anti-psychotic drugs (both typical anti-psychotic drugs and atypical neuroleptic drugs), antidepressants, anti-convulsants, anxiolytics, inhibitors of brain derived enzymes and the like.

Typical neuroleptic drugs, which are also known as neuroleptic agents or neuroleptics, are classical anti-psychotic drugs that are widely used in the treatment of central nervous system psychotic diseases and disorders, such as, for example, schizophrenia. The anti-psychotic efficacy of neuroleptics is attributed to their ability to antagonize/block central dopamine receptors. The neuroleptic drugs are known as typical anti-psychotic drugs and include, for example, phenothiazines, amongst which are aliphatics (e.g., chlorpromazine), piperidines (e.g., thioridazine) and piperazines (e.g., fluphenazine); butyrophenones (e.g., haloperidol); thioxanthenes (e.g., flupenthixol); oxoindoles (e.g., molindone); dibenzoxazepines (e.g., loxapine) and diphenylpiperidines (e.g., pimozide).

Unfortunately, the administration of neuroleptics is generally accompanied by adverse side effects which particularly include extrapyramidal symptoms, which are manifested as rigidity, tremor, bradykinesia (slow movement), and bradyphrenia (slow thought), as well as tardive dyskinesia, acute dystonic reactions and akathisia.

A different class of anti-psychotic drugs includes the atypical anti-psychotics. Atypical anti-psychotic drugs have a receptor binding profile that includes binding to central serotonin 2 receptors (5-HT2) in addition to dopamine D2 receptors. Atypical anti-psychotic drugs include, for example, clozapine, olanzapine and risperidone, and are generally characterized by high anti-serotonin activity and relatively low affinity to dopamine D2 receptors. Some atypical anti-psychotic drugs, such as clozapine, are known to further antagonize adrenergic, cholinergic and histaminergic receptors.

Unlike the neuroleptics, atypical anti-psychotics cause minimal extrapyramidal symptoms and thus rarely cause tardive dyskinesias, akathisia or acute dystonic reactions. However, the administration thereof involves other side effects such as increase of body weight, diabetes, elevated lipid level, mood disturbances, sexual disfunction, sedation, orthostatic hypotension, hypersalivation, lowered seizure threshold and agranulocytosis.

The severe side effects that are associated with both typical and atypical anti-psychotic drugs, which are collectively referred to herein as anti-psychotics, establish a major limitation to their use and extensive efforts have been made to develop anti-psychotic drugs devoid of these side effects.

Other psychotropic drugs are also typically associated with adverse side effects such as seizures, headaches, fatigue, hyperactivity, dizziness, orthostatic hypotension, dry mouth, sexual dysfunction, weight gain, prolonged QTc intervals, photosensitivity, restless leg syndrome, sedation and many more, which oftentimes critically limit the use thereof. A comprehensive list of such side effects can be found, for example, in "The Merck Manual of Medical Information" (Merck & Co. Inc.).

Recent studies on the development of extrapyramidal symptoms as a result of treatment with anti-psychotic drugs, mainly neuroleptics, have suggested a mechanism that involves an imbalance in the dopaminergic receptors D1 and D2, which is further accompanied by decreased activity of the γ-aminobutyric acid (GABA) system in the brain.

GABA is an important inhibitory neurotransmitter in the brain, which is known to have mood stabilizing activity, anxiolytic activity and muscle relaxant activity, and is further known to be related to some central nervous system (CNS) disorders and diseases. Recent studies on extrapyramidal symptoms suggest that GABA agonists may be further used to reduce neuroleptic-induced side effects and thus have an additional therapeutic potential.

Previous studies have already suggested that GABA agonists can interfere with other brain neurotransmitters and, in particular, with the dopamine system. Thus, it was found that GABA agonists can antagonize the neuroleptic-induced increase of dopamine receptors sensitivity and are therefore capable of improving neuroleptic-induced dyskinesia [Lloyd, K.G. et al., Pharmacol. Biochem. Behav. 1983, 18, 957-66]. Furthermore, it was found that some known direct GABA agonists (e.g., muscimol and SL 76002) cause a biphasic effect on haloperidol-induced catalepsy, such that while low doses of the agonist inhibit the stereotypic catalepsy behavior, high doses of the agonist potentiate the haloperidol-induced catalepsy. Other studies have reported that GABA agonists have anti-convulsive activity [Capasso, A. et al., Eur. Neuropsychopharmacol. 1997, 7, 57-63].

The use of GABA agonists is limited since they include hydrophilic functional groups (e.g., a free carboxylic acid group and a free amino group) and therefore do not readily cross the BBB. However, it was found that chemical conjugation of such compounds with fatty amino acids or peptides could substantially facilitate their passage across the BBB [Toth, I., J. Drug Target. 1994, 2, 217-39].

WO 03/026563, WO 05/092392, and U.S. Patent Application No. 10/808,541, disclosed novel chemical conjugates of various psychotropic agents and various organic acids. These chemical conjugates of various psychotropic agents and various organic acids. These chemical conjugates were designed so as to reduce the side effects induced by the psychotropic drug, to enhance the therapeutic efficacy of the psychotropic drugs and/or to exert a synergistic anti-proliferative activity, particularly in the brain. As taught in these patent applications, some of the most potent conjugates are conjugates that comprise a GABA residue covalently linked to a psychotropic drug. Such conjugates were found highly active as psychotropic agents whereby the side effects that were associated with the psychotropic drug when administered alone were substantially minimized.

While the GABA conjugates taught in WO 03/026563 and in U.S. Patent Application No. 10/808,541 include a free amine group that is derived from the GABA residue, and are therefore characterized by chemical instability, these compounds, according to the teachings of the above-mentioned patent applications, were prepared and practiced in the form of the HCl salt thereof.

However, as is well recognized in the art, HCl salts of amine-containing compounds may be unstable *ex-vivo* by being susceptible to rapid moisture absorption and degradation. These features pose some limitations to the preparation and storage of such salt forms and more importantly, to their use as pharmaceutical agents, which require high purity level.

Hence, while WO 03/026563 and U.S. Patent Application No. 10/808,541 teach highly beneficial conjugates of psychotropic drugs and organic acids, particularly GABA, the therapeutic use of such conjugates might be limited by the poor stability of conjugates that include a free amine group.

Thus, there is still a widely recognized need for, and it would be highly advantageous to have, conjugates of psychotropic drugs, and methods of preparing the same, which are characterized by improved therapeutic activity and reduced side effects, and which are further characterized by long-lasting *ex-vivo,* as well as *in-vivo* stability.

### SUMMARY OF THY INTENTION

According to one aspect of the present invention there is provided a chemical conjugate as defined in the annexed claims

According to further features in preferred embodiments of the invention described below, the chemical conjugate described herein is chemically stable under storage at a temperature that ranges from -50 °C and 50 °C for a time period of at least 7 days, and more preferably, for a time period of at least 14 days.

According to still further features in the described preferred embodiments a change in the purity of the chemical conjugate upon storage is less than 4 percents of the initial purity of the conjugate, and more preferably less than 1 percent of the initial purity of the conjugate.

According to still further features in the described preferred embodiments the chemical conjugate is characterized as non-hygroscopic such that a change in a water content of the chemical conjugate upon storage at a temperature that ranges from -50 °C and 50 °C is less than 0.4 weight percent of the total weight of the conjugate, and more preferably less than 0.2 weight percent of the total weight of the conjugate.

According to still further features in the described preferred embodiments the storage is for a time period of at least 14 days, and more preferably for a time period of at least 24 days.

According to still further features in the described preferred embodiments the second chemical moiety is covalently linked to the first chemical moiety via an ester bond selected from the group consisting of a carboxylic ester bond, an alkyloxy carboxylic ester bond, an amide bond and a thioester bond.

According to yet another aspect of the present invention there is provided a pharmaceutical composition comprising, as an active ingredient, the chemical conjugate described herein and a pharmaceutically acceptable carrier.

According to further features in preferred embodiments of the invention described below, the pharmaceutical composition is packaged in a packaging material and identified in print, on or in the packaging material, for use in the treatment of a psychotic disorder or disease, which may be schizophrenia.

The chemical conjugates in accordance with the invention can be used in method of treating a CNS disorder or disease in a subject, as presented herein, the method comprising administering to the subject a therapeutically effective amount of the chemical conjugate of the present invention.

According to further features in preferred embodiments of the invention described below, the chemical conjugate is administered intraperitoneally.

According to further features in preferred embodiments of the invention described below, the chemical conjugate is administered orally.

Accordingly, there is provided a use of the chemical conjugate described herein in the manufacture of a medicament, whereby the medicament can be for treating a psychotic disease or disorder, which may be schizophrenia.

According to a further aspect of the present invention there is provided a process of preparing a chemical conjugate of the present invention.

The process, according to this aspect of the present invention comprises:
providing an N-protected chemical conjugate including a first chemical moiety being covalently linked to a second chemical moiety, whereas the amino group is protected by an N-protecting group; removing the N-protecting group to thereby provide a free base form of the chemical conjugate; and contacting the free base form of the chemical conjugate with a first acid as defined in the annexed claims.

According to still further features in the described preferred embodiments removing the protecting group and the contacting are performed successively.

According to still further features in the described preferred embodiments removing the protecting group is effected by contacting the chemical conjugate with a second acid.

According to still further features in the described preferred embodiments the second acid is selected from the group consisting of trifluoroacetic acid and methanesulfonic acid.

According to still further features in the described preferred embodiments removing the protecting group and contacting the chemical conjugate with an acid are performed concomitantly without isolating the free base form of the conjugate.

According to further features in preferred embodiments of the invention presented below, the process further comprising, subsequent to contacting the chemical conjugate with an acid, adding an anti-solvent to thereby precipitate the addition salt of the chemical conjugate.

According to still further features in the described preferred embodiments, the anti-solvent is selected from the group consisting of hexane, heptane, octane, benzene, toluene, xylene and any mixture thereof.

According to still further features in the described preferred embodiments removing and contacting the N-protected chemical conjugate and the first acid are performed in the presence of a solvent, which is selected such that the first acid and the N-protected chemical conjugate are dissolvable therein and the chemical conjugate is precipitated therefrom.

According to further features in preferred embodiments of the present invention, the acid addition salts of the chemical conjugates have a purity that equals to or is greater than 97 percents.

According to further features in preferred embodiments of the invention described below, providing the N-protected chemical conjugate comprises reacting the anti-psychotic drug with an N-protected organic acid, as is detailed hereinbelow. According to a further aspect of the present invention there is provided a further process of preparing a chemical conjugate in accordance with the present invention.

The process, according to this aspect of the present invention comprises:
reacting the organic acid with an N-protecting group, to thereby obtain an N-protected organic acid; reacting the N-protected organic acid with the psychotropic drug; and removing the N-protecting group, thereby obtaining the conjugate in a free base form thereof.

According further features in preferred embodiments of the invention described below, the process further comprises, prior to reacting the anti-psychotic drug with an N-protected organic acid, reacting the N-protected organic acid with an acyl halide to thereby obtain a mixed anhydride derivative of the N-protected organic acid. Preferably, the acyl halide is selected from the group consisting of pivaloyl chloride, acetyl chloride, isobutyryl chloride and 3,3-dimethyl-butyryl chloride.

According to still further features in the described preferred embodiments reacting the N-protected organic acid with the acyl halide is performed in the presence of an organic base.

According to still further features in the described preferred embodiments reacting the mixed anhydride derivative of the N-protected organic acid with the anti-psychotic drug is performed during a time period that ranges from about 10 hours to about 20 hours.

According to still further features in the described preferred embodiments reacting the mixed anhydride derivative of the N-protected organic acid with the anti-psychotic drug is performed at a temperature lower than 50 °C, preferably at room temperature or a slightly elevated temperature.

According to still further features in the described preferred embodiments a molar ratio of the organic base and the N-protected organic acid ranges from about 1.3:1 to about 1:1.

According to still further features in the described preferred embodiments reacting the organic acid with the acyl halide is performed in the presence of a solvent. Preferably, the solvent is tetrahydrofurane (THF).

According to still further features in the described preferred embodiments reacting the anti-psychotic drug with an N-protected organic acid is performed in the presence of a solvent, an organic base and a dehydrating agent. Preferably the solvent is dichloromethane; preferably the organic base is selected from the group consisting of triethylamine, 4 dimethylaminopyridine, diethylamine, N-methylmorpholine and piperidine; and preferably the dehydrating agent is selected from the group consisting of N,N'-dicyclohexylcarbodiimide (DCC), N,N'-diisopropylcarbodiimide (DIC), 1-ethyl-3-(3-dimethylaminopropyl) carbodiimide (EDC) and N,N`-carbonyldiimidazole (CDI).

According to still further features in the described preferred embodiments the reaction is performed during a time period which ranges from about 2 hours to about 6 hours.

According to still further features in the described preferred embodiments the reaction is performed at a temperature lower than 50 °C, preferably at room temperature or a slightly elevated temperature.

According to still further features in the described preferred embodiments a molar ratio of the organic base and the N-protected organic acid ranges from about 0.1:1 to about 0.5:1.

According to still further features in the described preferred embodiments the process further comprises, prior to the reaction, mixing the anti-psychotic drug, the organic base and the N-protected organic acid at a temperature that ranges from about 0 °C to about 5 °C, to thereby obtain a slurry; adding the dehydrating agent to the slurry; and allowing the slurry to warm to room temperature.

According to still further features in the described preferred embodiments the reaction of the N-protected organic acid with the anti-psychotic drug is performed over a time period that ranges from about 8 hours to about 12 hours.

According to still further features in the described preferred embodiments the N-protecting group is selected from the group consisting of benzyloxycarbonyl (CBz), t-butoxycarbonyl (t-BOC), fluorenylmethoxycarbonyl (Fmoc), phthalimide (Pht) and benzenesulfonyl (Ts).

The present invention successfully addresses the shortcomings of the presently known configurations by providing novel acid addition salt forms of chemical conjugates of the anti-psychotic drug, which are characterized by improved stability and can therefore be beneficially utilized in the treatment of a variety of medical conditions and by further providing a novel process of preparing salts of such chemical conjugates, as well as the corresponding free base form of these conjugates.

Unless otherwise defined, all technical and scientific terms used herein have the same meaning as commonly understood by one of ordinary skill in the art to which this invention belongs

The term "comprising" means that other steps and ingredients that do not affect the final result can be added. This term encompasses the terms "consisting of" and "consisting essentially of".

The phrase "active ingredient" refers to a pharmaceutical agent including any natural or synthetic chemical substance that subsequent to its application has, at the very least, at least one desired pharmaceutical or therapeutic effect.

As used herein, the singular form "a", "an", and "the" include plural references unless the context clearly dictates otherwise. For example, the term "a compound" or "at least one compound" may include a plurality of compounds, including mixtures thereof.

Throughout this disclosure, various aspects of this invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

### DESCRIPTION OF THE PREFERRED EMBODIMENTS

The present invention is of novel salt forms of chemical conjugates of anti-psychotic drugs and organic acids, pharmaceutical compositions containing same and uses thereof in the treatment of psychotic disorders and diseases as defined in the annexed claims. The present invention is further of novel and improved processes of preparing acid addition salts of chemical conjugates of anti-psychotic drugs and organic acids which may involve preparing the corresponding free base forms thereof as defined in the annexed claims.

The principles and operation of the chemical conjugates, the processes, the compositions and the methods according to the present invention may be better understood with reference to the accompanying descriptions.

As discussed in detail in WO 03/026563 and U.S. Patent Application No. 10/808,541, chemical conjugates covalently coupling a psychotropic drug (which may have also anti-proliferative and/or chemosensitization activity) and an organic acid, preferably being a GABA agonist or an anti-proliferative agent, were found to exert high psychotropic and/or anti-proliferative therapeutic activity, as well as chemosensitization activity, associated with minimized adverse side effects. Some of the most potent conjugates disclosed in WO 03/026563 and U.S. Patent Application No. 10/808,541 are conjugates coupling a psychotropic drug and GABA.

However, as is discussed in detail hereinabove, while GABA is an organic acid that includes a free amine group, the free-base (amine) form of GABA-containing conjugates of psychotropic drugs as well as the HCl salt thereof are characterized by relative instability which limits the preparation and storage of such conjugates as well as the therapeutic use and efficacy thereof. As is demonstrated in the Examples section that follows, the free base form and the hydrochloric acid addition salt of an exemplary chemical conjugate of perphenazine and GABA, were found to be hygroscopic and hence unstable, rapidly absorbing water and breaking down to perphenazine, GABA and other substances, and therefore require special preparation and storage conditions.

While the high hygroscopicity of such chemical conjugates adversely affects the preparation, storage and resulting purity of the compounds, the instability of the conjugates, which is manifested by degradation to the chemical moieties from which the conjugates are composed, namely, the psychotropic drug and the organic acid (e.g., GABA), adversely affects the BBB permeability of the conjugate and particularly that of GABA, as well as the therapeutic effect of the conjugates, particularly in terms of reducing the side effects induced by the psychotropic drugs. Such instability may further adversely affect other pharmacokinetic and therapeutic parameters of the conjugates.

The instability of the chemical conjugates disclosed in WO 03/026563 and U.S. Patent Application No. 10/808,541, and particularly the instability of such conjugates which include a free amine group, as in the case of GABA-containing conjugates and other amino-containing organic acids, may therefore limit their otherwise highly beneficial therapeutic effect.

In a search for chemical conjugates of anti-psychotic drugs which would be characterized by improved *ex-vivo* and *in-vivo* performance, and while considering the high therapeutic efficacy of such chemical conjugates that include an amino-containing organic acid such as GABA, coupled to the anti-psychotic drug, the present inventors have designed and successfully prepared novel chemical conjugates of anti-psychotic drugs, in which the anti-psychotic drug is coupled to an organic acid that includes an amine group, such as GABA, whereby the amine group is in a form of an acid addition salt thereof. While conceiving the present invention, it was hypothesized that such acid addition salts of the chemical conjugates would be characterized, in addition to the beneficial therapeutic effect of the conjugates, by high stability and non-hygroscopicity and thus that the limitations associated with the presently known conjugates would be circumvented.

As is demonstrated in the Examples section that follows, while reducing the present invention to practice, various acid addition salts of GABA-containing conjugates of anti-psychotic drugs have been successfully prepared and were found both highly stable and non-hygroscopic, while maintaining the therapeutic advantages thereof.

Hence, according to one aspect of the present invention, there are provided novel chemical conjugates. Each of these chemical conjugates comprises a first chemical moiety, which is a anti-psychotic drug residue, covalently linked to a second chemical moiety, which is an amino-containing organic acid residue as defined in the annexed claims. The second chemical moiety is selected so as to reduce side effects that are induced by the anti-psychotic drug when administered *per se,* to enhance the therapeutic activity of the anti-psychotic drug and/or to exert anti-proliferative activity. In each of the chemical conjugates according to the present invention, the amino group in the organic acid residue is in the form of an acid addition salt thereof. Hydrochloric acid addition salts of such amine groups are excluded from the scope of this aspect of the present invention.

As used herein, the term "chemical moiety" refers to a residue derived from a chemical compound, which retains its functionality.

The term "residue" refers herein to a major portion of a molecule which is covalently linked to another molecule, as is well accepted in the art.

Thus, the phrase "anti-psychotic drug residue" refers to a major portion of a anti-psychotic drug as defined hereinbelow, which is covalently linked to another chemical moiety, as this term is defined hereinabove.

The phrase "organic acid residue" refers to a residue, as defined wherein, that is derived from an organic acid that includes a free carboxylic group.

The term "free carboxylic group" describes a -C(=O)OH group either as is, in its protonated or in its ionized or salt state.

The phrase "amino-containing organic acid residue" describes a residue, as defined herein, that is derived from an organic acid that includes a free carboxylic group, whereby the organic acid residue further includes a free amine group. This phrase is also referred to herein, interchangeably, as "an organic acid residue containing an amino group".

The terms "amine" and "amino", which are used herein interchangeably, describe a NR₁R₂R₃ group, where each of R₁, R₂ and R₃ is independently hydrogen, alkyl, cycloalkyl, aryl, heteroalicyclic and heteroaryl, as these terms are defined hereinbelow.

As is well known in the art, the phrase "acid addition salt" describes a complex of two ionizable moieties, a base and an acid, which, when interacted in a particular stoichiometric proportion and under suitable conditions, form a salt that comprises one or more cations of the base moiety and one or more anions of the acid moiety. As used herein, the phrase "acid addition salt" refers to such a complex as described hereinabove, in which the base moiety in amine, as defined hereinbelow, such that the salt comprises a cationic form of the amine and an anionic form of an acid.

Depending on the stoichiometric proportions between the base and the acid in the salt complex, as is detailed hereinbelow, the acid additions salts can be either mono addition salts or poly addition salts.

The phrase "mono addition salt", as used herein, refers to a salt complex in which the stoichiometric ratio between the acid anion and amine cation is 1:1, such that the acid addition salt includes one molar equivalent of the acid per one molar equivalent of the conjugate.

The phrase "poly addition salt", as used herein, refers to a salt complex in which the stoichiometric ratio between the acid anion and the amine cation is greater than 1:1 and is, for example, 2:1, 3:1, 4:1 and so on, such that the acid addition salt includes two or more molar equivalents of the acid per one molar equivalent of the conjugate.

The stoichiometric proportions between the base and the acid of the salt complex, according to preferred embodiments of the present invention, preferably range from 6:1 to 1:6 base:acid equivalents, more preferably from 4:1 to 1:4 base:acid equivalents, more preferably from 3:1 to 1:3 base:acid equivalents and more preferably from 1:1 to 1:3 base:acid equivalents.

The acid addition salts of a chemical conjugate according to the present invention are therefore complexes formed between one or more amino groups of the drug and one or more equivalents of an acid.

The acid addition salts may include maleic acid which affords a maleic acid addition salt, methanesulfonic acid which affords a methanesulfonic acid (mesylate) addition salt and oxalic acid which affords an oxalic acid addition salt. Each of these acid addition salts can be either a mono acid addition salt or a poly acid addition salt, as these terms are defined hereinabove.

As is demonstrated in the Examples section that follows, chemical conjugates according to the present invention, which include the mono or poly maleaic acid addition salt, the mono or poly methanesulfonic acid addition salt, and the mono or poly oxalic acid addition salt of the amine group in a perphenazine-GABA conjugate, were successfully prepared both in small-scale and large-scale processes. These chemical conjugates were found highly chemically stable and non-hygroscopic under various conditions, particularly in comparison with the corresponding free base form and HCl addition salt form of a perphenazine-GABA conjugate.

The phrase "chemically stable", as used herein, describes an attribute of a chemical substance which is characterized by low susceptibility to chemical and physical reactions such as, for example, decomposition, degradation, conjugation, polymerization, oxidation or any other alterations, which may lead to chemical changes of a substantial portion of the substance and thus may substantially affect its purity, over time. By "substantial portion" it is meant more than 5 percents and even more than 2 percents of the substance. In other word, this phrase describes an attribute of a chemical substance which remains chemically unchanged over time. By "over time" it is meant a relative time period (e.g., from a few hours to a few weeks) at which the substance remains chemically unchanged when kept under various conditions, as is detailed hereinunder.

The chemical stability of the chemical conjugates of the present invention is particularly reflected by the tendency of the conjugates to undergo degradation into the chemical moieties from which the compounds are composed, that is, the anti-psychotic drug and the organic acid. As discussed hereinabove, such degradation adversely affects the pharmacological purity of the conjugates, as well as the therapeutic efficacy thereof.

Thus, as is demonstrated in the Examples section that follows, the above-mentioned exemplary conjugates according to the present invention were found highly stable when kept under various conditions. The various conditions were reflected by the atmosphere in the container in which the conjugates were kept, the seal of the container, the temperature at which the conjugates were kept and/or the tested time period.

The chemical stability of the conjugates was determined by HPLC analyses and was measured by the relative peak areas of the substance and of at least one of its degradation products. Thus, as is further exemplified in the Examples section that follows, the chemical stability of the exemplary conjugates described above was measured during time periods that typically ranged from 1 day to 24 days, whereby the conjugates were kept in sealed or open containers, with or without nitrogen atmosphere, and at a temperature range of from -20 °C to 40 °C. As is detailed in the Examples section that follows, the conjugates remained chemically unchanged for prolonged time periods of more than two weeks, at least when kept in a sealed container at low temperatures, such than the sum of all the impurities including, for example, perphenazine, that were formed during the tested time period, did not exceed 4 percents, and mostly did not exceed 2 percents of the original purity of the conjugates, as determined by HPLC analysis.

Hence, according to an embodiment of the present invention, the chemical conjugates described herein are chemically stable for a time period of at least 7 days, preferably at least 10 days, more preferably at least 14 days, more preferably at least 24 days, and up to a few months, when kept at a temperature of from about -50 °C to about 50 °C.

As used herein the term "about" refers to ± 10 %.

As discussed hereinabove, such a chemical stability was observed when the conjugates were kept at such temperatures either in an open container, a sealed container and a sealed container having a nitrogen atmosphere.

As is further exemplified in the Examples section that follows, the chemical conjugates according to the present invention were found non-hygroscopic.

The term "non-hygroscopic" as used herein refers to an attribute of a chemical substance, which does not exhibit a substantial change in its water content over time (*vide supra*). By "substantial change" it is meant a change of more than 5 percents and even of more than 1 percent in the water content of the substance.

As is demonstrated in the Examples section that follows, the conjugates according to the present invention were tested for the hygroscopicity thereof upon storage for various time periods under various conditions, as detailed hereinabove. It was found that the change in the water content of the conjugates, at least when kept in a sealed container at low temperature, and in some cases when kept at higher temperatures and/or open container, was less than 1 percent, and even less than 0.2 percent, as determined by HPLC analyses.

The non-hygroscopicity characteristic of the chemical conjugates described herein is highly beneficial in terms of ease of handling and storage of the chemical conjugates. This characteristic though may further provide for the improved stability of the chemical conjugates since it is postulated that one of the factors that affect the decomposition and degradation of chemical conjugates of anti-psychotic drugs and organic acids is the high water absorption thereof, which leads to hydrolysis of the bond coupling these two moieties and/or to de-esterification in cases of an ester coupling bond. It should be noted, however, that other factors, such as, for example, a higher free energy barrier for chemical interaction, attribute to the improved stability of the chemical conjugates of the present invention.

In each of the chemical conjugates described herein, the organic acid residue, according to the present invention and as is detailed in WO 03/026563 and U.S. Patent Application No. 10/808,541, is selected so as to reduce the side effects that could be induced by the anti-psychotic drug if administered alone, to enhance the therapeutic efficacy of the anti-psychotic drug by providing as added therapeutic value to the chemical conjugate.

The second chemical moiety in the chemical conjugates of the present invention is a GABA agonist residue.

As used herein, the phrase "GABA agonist residue" refers to a residue, as this term is defined hereinabove, of a GABA agonist, while the term "GABA agonist" describes compounds that are capable of activating the GABA system in the brain either directly or indirectly, including compounds that directly bind the GABA receptor or to any other receptor that affects the GABA system and are therefore pharmacologicallly related to GABA. The term "GABA agonist" is hence understood to refer to GABA itself, whereas the term "GABA agonist residue" is hence understood to refer to a residue of GABA.

As described hereinabove, in each of the chemical conjugates described herein, the second chemical moiety is covalenty coupled to a anti-psychotic drug residue. The psychotropic drug residue, according to the present invention, is a perphenazine residue.

The second chemical moiety in the chemical conjugates of the present invention is covalently linked to the first chemical moiety preferably via an ester bond. The ester bond can be a carboxylic ester bond, an oxyalkyl carboxylic ester bond, an amide bond or a thioester bond.

As used herein, the phrase "carboxylic ester bond" includes an "-O-C(=O)-" bond.

As used herein, the phrase "oxyalkyl carboxylic ester bond" includes an "O-R-O-C(=O)-" bond, where R is an alkyl, as defined hereinabove. Preferably R is methyl.

The phrase "amide bond" includes a "-NH-C(=O)-" bond.

The phrase "thioester bond" includes a "-S-C(=O)-" bond.

Such ester bonds are known to be hydrolizable by brain derived enzymes, such as esterases and amidases, and hence the chemical conjugates of the present inventions can act as prodrugs that are metabolized in the brain and thereby simultaneously release the anti-psychotic and the organic acid, thus, providing for advantageous co-pharmacokinetics for the anti-psychotic and the organic acid.

This feature is highly advantageous since it provides (i) a simultaneous action of the anti-psychotic drug and the organic acid, which synergistically results in reduced side effects induced by the drug and in dual activity of both moieties; (ii) higher affinity of the prodrug to the dopaminergic receptors which results in synergistically higher anti-psychotic activity and synergistically higher anti-proliferative activity toward brain proliferative disorders; and (iii) improved brain permeability of both chemical moieties.

According to another aspect of the present invention, there is provided a process of preparing the chemical conjugates presented herein namely, a process of preparing the acid addition salts of chemical conjugates which include two chemical moieties being covalently linked to one another, wherein one chemical moiety is a anti-psychotic drug residue and the other chemical moiety is an organic acid residue containing an amino group as defined in the annexed claims.

The process, according to this aspect of the present invention, is generally effected by providing an N-protected form of the chemical conjugate (in which the amine group of the second moiety is protected by an N-protecting group); removing the N-protecting group, to thereby provide a free base form of the chemical conjugate; and contacting this free base form of the chemical conjugate with an acid, referred to herein as the first acid, to thereby provide the acid addition salt of the chemical conjugate.

Acids that are suitable for use a the first acid in this context of the present invention are maleic acid, methanesulfonic acid and oxalic acid.

The phrase "N-protecting group" as used herein describes a chemical moiety which is covalently bound to the nitrogen atom of an amine group of a chemical substance, such that this amine group is rendered inactive, or blocked from reacting with other chemical species as long as the protecting group is attached thereto. The protecting group is selected such that it can be readily removed at certain well-known chemical and/or physical conditions, which do not affect other groups in the compound. Typically, each protecting group can be removed under conditions which are specific thereto. A suitable protecting group is therefore often selected upon these conditions, whereby these conditions determine the ability to safely remove the protecting group without affecting other chemical and structural features of the compound. Such chemical and/or physical conditions may be, for example, temperature, pH, pressure.

The removal of the N-protecting group, according to preferred embodiments of the present invention, is carried out under acidic conditions. Acidic conditions include, for example, an acidic environment which comprises an acid (organic or inorganic).

Non-limiting examples of N-protecting groups that are suitable for use in the context of the present invention include benzyloxycarbonyl (CBz), t-butoxycarbonyl (t-BOC), fluorenylmethoxycarbonyl (Fmoc), phthalimides (Pht) and benzenesulfonyl (Ts).

As discussed herein, the formation of an acid addition salt of the free-base form of the chemical conjugate occurs once the N-protecting group is removed from the amino group of the organic acid residue. Preferably, the removal of the protecting group is effected by means of reacting the N-protected conjugate with an acid. Once the protecting group has been removed, the resulting free base can be first isolated and/or be directly reacted with the first acid so as to obtain the final and desired addition salt.

Hence, according to preferred embodiments, removing the N-protecting group is effected prior to contacting the obtained free base form of the chemical conjugate with the first acid for the addition salt, such that the chemical conjugate, in its free base form, is first isolated and thereafter reacted with the first acid to form the acid addition salt successively.

According to another preferred embodiment of the present invention, the removal of the protecting group can be effected by contacting the N-protected chemical conjugate with a second acid, such as, but not limited to, trifluoroacetic acid or methanesulfonic acid. Once deprotection is effected by the second acid, the second acid is quenched and removed and the free-base form of the chemical conjugate is isolated. Thereafter the first acid is introduced so as to form the acid addition salt.

As discussed herein, the free base form of the chemical conjugates described herein may be highly unstable, as demonstrated for the perphenazine-GABA conjugate in the Examples section that follows. Hence, alternatively, the removal of the protecting group is effected concomitantly with the formation of the addition salt, such that the acid used for the deprotection procedure also serves as the first acid that forms the final desired addition salt, without isolating the free base form of the chemical conjugate.

Thus, according to still another preferred embodiment, removing the N-protecting group and contacting the obtained free base with the acid of the addition salt are effected concomitantly, more preferably under the same reaction conditions and in the same reaction pot, namely, *in-situ.*

The acid that is suitable to prepare addition salts of the chemical conjugates according to this embodiment of the present invention, in the concomitant deprotection and salt formation, is methanesulfonic acid.

Removing the N-protecting group and/or reacting the free base form of the conjugate with the first acid are preferably performed under such conditions that would not affect the structural and chemical features of the conjugate. Special attention is taken regarding the bond linking the psychotropic drug residue and the organic acid residue. Thus, preferably, the process of preparing the acid addition salt is performed under such conditions that would not lead to the formation of degradation products of the conjugate. These include, for example, dry nitrogen atmosphere, low temperatures (lower than 50 °C), as is detailed hereinunder. The appearance of degradation products is preferably monitored (e.g., by HPLC) during the process.

Solvent effect studies were carried out to find the most suitable solvent which will effect the precipitation of the acid addition salt (the desired product). These studies determined that amongst the tested solvents, suitable solvents include dichloromethane, acetonitrile and THF, whereby the presently most suitable solvent, according to the present embodiments, is acetonitrile.

The process of the concomitant deprotection and addition salt formation may further include the addition of an anti-solvent, which serves to promote the precipitation of the acid addition salt and thus further to drive the reaction towards completion. Exemplary anti-solvents that are suitable for use in this context of the present embodiments include, without limitations, hexane(s), heptane(s), octane(s), benzene, toluene, xylene(s) and any mixture thereof. Preferably, the anti-solvent is heptane and/or toluene.

The process of the concomitant deprotection and addition salt formation may optionally be performed in such a solvent, in which the N-protected chemical conjugate and the first acid are both dissolvable, whereby during the reaction, the formed acid addition salt product precipitates out of the solution, thereby driving the reaction towards completion. Exemplary suitable solvents include, without limitation, acetone, alkyl acetate (e.g., isobutyl acetate) and a combination thereof.

As is demonstrated in the Examples section that follows, using the process described above, the chemical conjugates described herein are obtained in a purity that is equal or greater than 97 %, preferably greater than 98.5 %, as determined by HPLC analyses, and a yield that is equal or greater than 70 %, preferably greater than 77 %.

In the course of preparing the novel acid addition salts of the chemical conjugates presented herein, novel and improved synthetic pathways have been developed for preparing the conjugates of anti-psychotic drugs and organic acids, initially described in WO 03/026563 and U.S. Patent Application No. 10/808,541. These newly developed processes further served for preparing an amino-protected form of the conjugates of psychotropic drugs and amino-containing organic acids which were used for preparing the various addition salts described hereinabove, including hydrochloric acid (HCl) addition salts of these conjugates.

Thus, according to an additional aspect of the present invention, there is provided a process of preparing a chemical conjugate which comprises a anti-psychotic drug residue and an organic acid residue that is selected so as to exert additional therapeutic effects over that of the anti-psychotic drug, enhance its efficacy and reduce side-effects thereof. The process, according to this aspect of the present invention, can be utilized for preparing any of the conjugates described in WO 03/026563 and U.S. Patent Application No. 101/808,541, and is particularly useful for preparing such conjugates in which the organic acid residues comprises an amino group. This process is generally effected by converting the organic acid to a reactive intermediate/derivative thereof and reacting this reactive intermediate/derivative with the anti-psychotic drug, in the presence of a solvent and an organic base.

The chemical conjugates obtained by the process according to this aspect of the present invention, in which the organic acid is an amino-containing organic acid, are in a free base form thereof. The process disclosed herein was designed so as to perform this reaction under mild conditions, particularly as compared with the process described in WO 03/026563 and U.S. Patent Application No. 10/808,541.

During the coupling reaction, a bond is formed between the carboxylic acid group of the organic acid, and a functional group of the anti-psychotic drug, being, for example, a hydroxyl, a thiol or an amine, so as to form, for example, an ester bond in case of a hydroxyl, a thioester in case of a thiol and an amide in case of an amine, between the organic acid residue and the anti-psychotic drug residue.

The process for preparing the chemical conjugates according to this aspect of the present invention is based on two approaches, as follows:
One approach is based on reacting the organic acid, preferably an N-protected amino-containing organic acid, with quantitative amounts of an acyl halide in the presence of an organic base so as to form a highly reactive mixed anhydride derivative thereof, and thereafter reacting this mixed anhydride derivative with the anti-psychotic drug, so as to obtain the chemical conjugate, preferably in an N-protected form thereof.

According to preferred embodiments of the present invention, the acyl halide is selected from the group consisting of pivaloyl chloride, acetyl chloride, isobutyryl chloride and 3,3-dimethyl-butyryl chloride, and more preferably the acyl halide is pivaloyl chloride.

The presence of the organic base is aimed at promoting the anhydride and conjugate formation reactions and leading to the reactions completion. Hence, the molar ratio of the organic base and the organic acid preferably ranges from about 2:1 to about 1:1, more preferably from about 1.5:1 to about 1:1, more preferably from about 1.3:1 to about 1:1, and is preferably about 1:1. Thus, the organic base is preferably added in quantitative amounts with respect to the starting organic acid and anti-psychotic drug.

The conjugation reaction can be carried out in a solvent such as, for example, tetrahydrofurane (THF), preferably at room temperature, or otherwise at a slightly elevated temperature, preferably lower than 50 °C, and over a time period which ranges from about 10 hours to about 20 hours.

Exemplary organic bases that are suitable for use in this context of the present invention include triethylamine, 4-dimethylaminopyridine, diethylamine, N-methylmorpholine and piperidine. Preferably the organic base is triethylamine.

In another approach, reacting the organic acid and the anti-psychotic drug is performed in the presence of a dehydrating (coupling) agent, a solvent, and an organic base.

Reacting the organic acid with the anti-psychotic drug is preferably performed at room temperature, or otherwise at a slightly elevated temperature, preferably lower than 50 °C.

The yields and purity of the product were compared with those obtained by the process described in WO 03/026563 and U.S. Patent Application No. 10/808,541, in which DMF was used as the solvent, to evaluate the solvent effect. It was found that dichloromethane is a more suitable solvent for perphenazine and a better anti-solvent for the formed byproduct 5,6-dihydrouracil (DHU) which readily precipitated in dichloromethane as a fine free flowing solid. Thus, in preferred embodiments, the solvent is dichloromethane.

The phrase "dehydrating agent", as used herein, describes a reagent which promotes a coupling reaction in which one or more water molecules are released. Dehydration agents typically act by reducing the concentration of the released water molecules in the reaction mixture, to thereby drive the reaction towards products formation. Exemplary dehydrating agents that are suitable for use in this context of the present invention include, without limitation, N,N'-dicyclohexylcarbodiimide (DCC), N,N'-diisopropylcarbodiimide (DIC), 1-ethyl-3-(3-dimethylaminopropyl) carbodiimide (EDC) and N,N'-carbonyldiimidazote (CDI).

The organic base is used in this process in catalytic amounts with respect to the starting N-protected amino acid and anti-psychotic drug. Hence, preferably, the molar ratio of the organic base and the organic acid ranges from about 0.05:1 to about 0.5:1, more preferably from about 0.1:1 to about 0.5:1, and more preferably is about 0.3:1.

Exemplary organic bases that are suitable for use in this context of the present invention include, without limitation, triethylamine, 4-dimethylaminopyridine, diethylamine, N-methylmorpholine and piperidine. Preferably the organic base is 4-dimethylaminopyridine.

The dehydrating agent-driven coupling reaction is preferably carried out during a time period which ranges from 2 hours to 6 hours.

Alternatively, a mixture of the anti-psychotic drug, the organic base and the organic acid is prepared in a form of a slurry prior to the coupling reaction. Preferably, this slurried mixture is prepared at a temperature that ranges from about 0 °C to 5 °C. The dehydrating agent is thereafter added portion-wise to the slurry, and the resulting mixture is allowed to warm to room temperature. Such a coupling reaction can be completed overnight while agitating the mixture at room temperature.

Using the process presented above, the chemical conjugates are obtained in higher yield and purity, particularly as compared to conjugates obtained by the process described in WO 03/026563 and U.S. Patent Application No. 10/808,541. Depending on the selected approach, the yield may be as high as 90 % for the mixed anhydride approach, and as high as 98 % yield for the dehydrating agent approach. Preferably, the purity of the product obtained by the present process, as determined by HPLC, is equal to or higher than 98 %, more preferably the purity is equal to or higher than 99 % and even equal to or higher than 99.5 %.

These new and efficient synthetic approaches for preparing the chemical conjugates in a free base form thereof, which are presented and demonstrated in detail in the Examples section that follows, and exemplified by Examples 3, 4 and 5, were used to prepare the starting N-protected chemical conjugate which was subsequently used in the preparation of the novel addition salts of the chemical conjugates presented herein.

Any of the processes presented hereinabove can be utilized to prepare hydrochloric acid addition salts thereof. A hydrochloric acid addition salt can be obtained by reacting an N-protected chemical conjugate, preferably prepared as described herein, with hydrochloric acid, whereby the reaction can be effected by any one of the processes for preparing an addition salt which are presented hereinabove, or by the process described in WO 03/026563 and U.S. Patent Application No. 10/808,541.

Further according to the present invention there is provided a pharmaceutical composition which comprises the chemical conjugate of the invention, as an active ingredient, and further comprises a pharmaceutically acceptable carrier.

As used herein a "pharmaceutical composition" refers to a preparation of one or more of the chemical conjugates described herein, with other chemical components such as pharmaceutically suitable carriers and excipients. The purpose of a pharmaceutical composition is to facilitate administration of a compound to a subject.

Hereinafter, the term "pharmaceutically acceptable carrier" refers to a carrier or a diluent that does not cause significant irritation to a subject and does not abrogate the biological activity and properties of the administered compound. Examples, without limitations, of carriers are propylene glycol, saline, emulsions and mixtures of organic solvents with water.

Herein the term "excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

In one embodiment of the present invention, the pharmaceutical composition is formulated as a solution, for administration by injection. According to this embodiment, the pharmaceutical carrier can be, for example, an aqueous solution of lactic acid, e.g., a 1 % solution of lactic acid.

In another embodiment of the present invention, the pharmaceutical composition is formulated for oral administration, either as a solution, as described hereinabove, or as a solid dosage form. When formulated for oral administration, the pharmaceutical composition can be in a form of, for example, capsules, pills and tablets, as is detailed hereinunder.

In this respect; it should be pointed out that some of the chemical conjugates of the present invention, according to preferred embodiments, are readily soluble in aqueous media and are therefore easily formulated. Such convenient formulation provides an additional advantage of the chemical conjugates of the present invention over the known conjugates of anti-psichotic drugs, which typically include long-chain fatty acids and are therefore non-soluble in aqueous media and administered as oily formulation.

Techniques for formulation and administration of drugs may be found in ``Remington's Pharmaceutical Sciences," Mack Publishing Co., Easton, PA, latest edition.

Suitable routes of administration may, for example, include oral, rectal, transmucosal, transdernal, intestinal or parenteral delivery, including intramuscular, subcutaneous and intramedullary injections as well as intrathecal, direct intraventricular, intravenous, intraperitoneal, intranasal, or intraocular injections. Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more pharmaceutically acceptable carriers comprising excipients and auxiliaries, which facilitate processing of the active compounds into preparations which can be used pharmaceutically. Proper formulation is dependent upon the route of administration chosen.

For injection, the chemical conjugates of the invention may be formulated in aqueous solutions, preferably in physiologically compatible buffers such as Hank's solution, Ringer's solution, or physiological saline buffer with or without organic solvents such as propylene glycol, polyethylene glycol. For transmucosal administration, penetrants are used in the formulation. Such penetrants are generally known in the art.

For oral administration, the chemical conjugates can be formulated readily by combining the active compounds with pharmaceutically acceptable carriers well known in the art. Such carriers enable the conjugates of the invention to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carbomethylcellulose and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions, which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by inhalation, the chemical conjugates for use according to the present invention are conveniently delivered in the form of an aerosol spray presentation from a pressurized pack or a nebulizer with the use of a suitable propellant, e.g., dichlorodifluoromethane, trichlorofluoromethane, dichlorotetrafluoroethane or carbon dioxide. In the case of a pressurized aerosol, the dosage unit may be determined by providing a valve to deliver a metered amount. Capsules and cartridges of, e.g., gelatin for use in an inhaler or insufflator may be formulated containing a powder mix of the compound and a suitable powder base such as lactose or starch.

The chemical conjugates described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active compound in water-soluble form. Additionally, suspensions of the active compounds may be prepared as appropriate oily injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the conjugates to allow for the preparation of highly concentrated solutions.

Alternatively, the active ingredient may be in powder form for constitution with a suitable vehicle, e.g., sterile, pyrogen-free water, before use.

The chemical conjugate of the present invention may also be formulated in rectal compositions such as suppositories or retention enemas, using, e.g., conventional suppository bases such as cocoa butter or other glycerides.

The pharmaceutical compositions herein described may also comprise suitable solid of gel phase carriers or excipients. Examples of such carriers or excipients include, calcium carbonate, calcium phosphate, various sugars, starches, cellulose derivatives, gelatin and polymers such as polyethylene glycols.

Pharmaceutical compositions suitable for use in context of the present invention include compositions wherein the active ingredients are contained in an amount effective to achieve the intended purpose. More specifically, a therapeutically effective amount means an amount of chemical conjugate effective to prevent, alleviate or ameliorate symptoms of disease or prolong the survival of the subject being treated.

Determination of a therapeutically effective amount is well within the capability of those skilled in the art, especially in light of the detailed disclosure provided herein.

For any chemical conjugate of the invention, the therapeutically effective amount or dose can be estimated initially from activity assays in cell cultures and/or animals. For example, a dose can be formulated in animal models to achieve a circulating concentration range that includes the IC50 as determined by activity assays (e.g., the concentration of the test compound, which achieves a half-maximal inhibition of the proliferation activity). Such information can be used to more accurately determine useful doses in humans.

Toxicity and therapeutic efficacy of the chemical conjugates described herein can be determined by standard pharmaceutical procedures in experimental animals, e.g., by determining the IC50 and the LD50 (lethal dose causing death in 50 % of the tested animals) for a subject compound. The data obtained from these activity assays and animal studies can be used in formulating a range of dosage for use in human.

The dosage may vary depending upon the dosage form employed and the route of administration utilized. The exact formulation, route of administration and dosage can be chosen by the individual physician in view of the patient's condition. (See, e.g., Fingl, et al., 1975, in "The Pharmacological Basis of Therapeutic", Ch. 1 p.1).

Dosage amount and interval may be adjusted individually to provide plasma levels of the active moiety which are sufficient to maintain the psychotropic and/or the anti-proliferative effects, termed the minimal effective concentration (MEC). The MEC will vary for each preparation, but can be estimated from *in vitro* and/or *in vivo* data, e.g., the concentration necessary to achieve 50-90 % inhibition of a proliferation of certain cells may be ascertained using the assays described herein. Dosages necessary to achieve the MEC will depend on individual characteristics and route of administration. HPLC assays or bioassays can be used to determine plasma concentrations.

Dosage intervals can also be determined using the MEC value. Preparations should be administered using a regimen, which maintains plasma levels above the MEC for 14-90 % of the time, preferable between 30-90 % and most preferably 50-94 %.

Depending on the severity and responsiveness of the condition to be treated, dosing can also be a single administration of a slow release composition described hereinabove, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as a FDA approved kit, which may contain one or more unit dosage forms containing the active ingredient The pack may, for example, comprise metal or plastic foil, such as a blister pack. The pack or dispenser device may be accompanied by instructions for administration. The pack or dispenser may also be accompanied by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a chemical conjugate of the invention formulated in a compatible pharmaceutical carrier may also be prepared, placed in an appropriate container, and labeled for treatment of an indicated condition. Suitable conditions are psychotic diseases and disorders such as schizophrenia, disease, Alzheimer disease and epilepsy, brain proliferative disorders and MDR cancer, and chemosensitization, as this term is defined hereinabove.

Hence, according to preferred embodiments of the present invention, the pharmaceutical composition described herein is packaged in a packaging material and is identified in print, on or in the packaging material, for use in the treatment of psychotic disorders or diseases.

The conjugates according to the present invention can be used in a method for treating or preventing a psychotic disorder or disease in a subject (e.g., a human being). The method is effected by administering a therapeutically effective amount of one or more of the chemical conjugates of the invention to a treated subject

As used herein, the term "method" refers to manners, means, techniques and procedures for accomplishing a given task including, those manners, means, techniques and procedures either known to, or readily developed from known manners, means, techniques and procedures by practitioners of the chemical, pharmacological, biological, biochemical and medical arts.

Herein, the term "treating" includes abrogating, substantially inhibiting, slowing or reversing the progression of a disease, substantially ameliorating clinical symptoms of a disease or substantially preventing the appearance of clinical symptoms of a disease.

The term "administering" as used herein refers to a method for bringing a chemical conjugate of the present invention into an area or a site in the brain that affected by the psychotropic disorder or disease.

The chemical conjugate of the present invention can be administered intraperitoneally. More preferably, it is administered orally.

The term "subject" refers to animals, typically mammals having a blood brain barrier, including human beings.

The term "therapeutically effective amount" refers to that amount of the chemical conjugate being administered which will relieve to some extent one or more of the symptoms of the psychotic disorder or disease being treated.

A therapeutically effective amount according to this aspect of the present invention preferably ranges between 0.01 mg/kg body and 50 mg/kg body, more preferably between 0.01 mg/kg body and 25 mg/kg body, more preferably between 0.05 mg/kg body and 10 mg/kg body and most preferably between 0.05 mg/kg body and 5 mg/kg body.

The present invention is thus directed to chemical conjugates which exert anti-psychotic activity. The chemical conjugates of the present invention are highly advantageous since they exert enhanced anti-psychotic activity and are further characterized by minimized adverse side effects induced thereby.

The term "side effect" as used herein refers to adverse symptoms that may develop as a result of administering to a subject a certain drug. Such symptoms may include, for example, extrapyramidal symptoms, as is detailed hereinabove, and are typically associated with the administration of antipsychotic drugs. Other side effects which are typically associated with psychotropic drugs include, for example, orthostatic hypotension, dry mouth, sexual dysfunction, weight gain, prolonged QTc intervals, photosensitivity, restless leg syndrome and sedation.

### EXAMPLES

Preference is now made to the following examples, which together with the above descriptions, illustrate the invention in a non limiting fashion.

### MATERIALS AND EXPERIMENTAL METHODS

All reagents and solvents were purchased from commercial vendors such as Aldrich, Sigma, Fluka and Merck.

AN-197 and AN-168 were prepared according to the procedures described in WO 03/026563, unless otherwise indicated.

HPLC analyses were performed on a Water 2695 Separations Module, using a Luna C18 (2) 50 mm x 4.6 mm x 3 um column, a 5 µl injection volume and a 2487 dual wavelength detector under the following instrumental conditions: Mobile phase A: 0.1 % formic acid; Mobile phase B: acetonitrile; Flow rate: 0.5 ml per minute; Mobile phase gradient: 0.0 minutes 90 % A, 10 % B, 14.0 minutes 20 % A, 80 % B, 15.0 minutes 90 % A, 10 % B and 20.0 minutes 90 % A, 10 % B; Run time: 20 minutes; Detection: UV at 254 nm; Column temperature: 40 °C.

NMR spectra were recorded using a Bruker-Avance 500 MHz device.

Water absorption and content measurements were carried out using a Karl Fischer (KF) titrator. Karl Fischer titration is a classic method in analytical chemistry that uses calometric titration to determine the moisture content of a sample, which is specific to water. The titration reaction taking place in the presence of a base and 50:50 methanol:dichloromethane as a titration solvent.

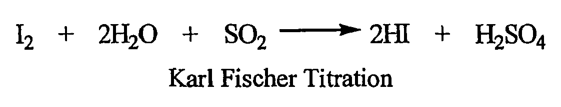

The Karl Fischer chemical reaction takes place between iodine and water with the reactants being in a 1 to 1 ratio between iodine and water.

The accurately weighted samples (near 100 mg) of the tested compound were placed in the titrator to determine the amount of sample required for measuring 10-250 µg of water, and the water content was calculated accordingly.

### CHEMICAL SYNTHESES, ANALYSES AND RESULTS

### REFERENCE EXAMPLE 1

### Synthesis of perphenazine N-Boc-4-aminobutyrate (AN-197) according to WO 03/026563

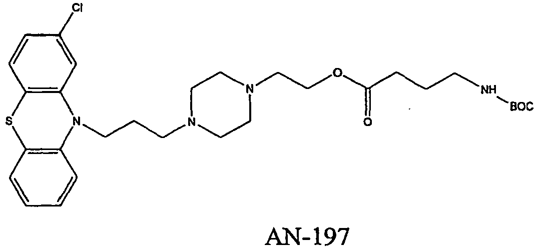

AN-197 was prepared as described in WO 03/026563 and U.S. Patent Application No. 10/808,541. In brief, a mixture of N-Boc-protected γ-aminobutyric acid (Sigma, Cat. No. 15294) (1 equivalent) and carbonyl diimidazole (CDI, Fluka, Cat. No. 21860) (1.1 equivalents) in 10 ml DMF (1 volume) was stirred, under nitrogen atmosphere, for 1 hour. Perphenazine (Sigma, Cat. No. P6402) (1 equivalent) was added thereafter and the mixture was stirred under nitrogen atmosphere, at 90°C, for 24 hours. The resulting slurry was evaporated and partitioned between ethyl acetate and water. The aqueous phase was extracted twice with ethyl acetate and the combined organic layer was washed trice with NaHCO₃, twice with brine, dried over MgSO₄, filtered and evaporated. The N-protected product was obtained as yellowish oil.

The crude product was purified by silica gel chromatography, using a mixture of 20:1 ethyl acetate:ethanol as eluent. The purified product was obtained as a yellowish oil (63 % yield).

The purity of the final product was tested and found to be 98.83 %, as determined by HPLC.

¹H-NMR (CDCl₃): δ = 1.43 (s, 9H, t-Bu), 1.82 (quint, J = 7.18 Hz, 2H, CH₂CH₂NHBoc), 1.90 (quint, J = 7.18 Hz, 2H, ArNCH₂CH₂), 2.35 (t, J = 8.97 Hz, 2H, CO₂CH₂), 2.42 (m, 10H, five NCH₂), 2.60 (t, J = 5.98 Hz, 2H, NCH₂CH₂O), 3.16 (q, J = 6.85 Hz, 2H, CH₂NHBoc), 3.84 (t, J = 7.2 Hz, 2H, ArNCH₂), 4.18 (t, J = 5.98 Hz, 2H, NCH₂CH₂O), 5.10 (bs, 1H, NH), 6.83 (m, 7H, Ar) ppm.

¹³C-NMR (CDCl3): δ = 23.92 (CH₂CH₂NHBoc), 24.98 (ArNCH₂CH₂), 28.21 (t-Bu), 39.50 (CH₂CO₂), 45.05 (ArNCH₂), 52.89 (two NCH₂), 53.03 (two NCH₂), 55.15 (ArNCH₂CH₂CH₂), 56.34 (NCH₂CH₂O), 60.13 (CH₂NHBoc), 61.29 (NCH₂CH₂O) 78.80 (CMe₃), 115.60 (C₁, C₁₀), 121.96 (C₃), 122.65 (C₈), 123.22 (C₅), 124.45 (C₆), 127.21 (C₇, C₄), 127.62 (C₉), 132.93 (C₂), 144.23 (C₁₂), 146.23 (C₁₁), 155.79 (NCO₂), 172.92 (CO₂) ppm.

### REFERENCE EXAMPLE 2

### Synthesis of perphenazine 4-aminobutyrate hydrochloride (AN-168) according to WO 03/026563

AN-168 was prepared by removing the N-protecting group from perphenazine N-Boc-4-aminobutyrate (AN-197), as described in WO 03/026563. Briefly, a solution of 4 N HCl in ethyl acetate was added dropwise to a solution of N-protected product (perphenazine N-Boc-4-aminobutyrate, AN-197) in ethyl acetate. The mixture was stirred for 2 hours at room temperature. The solvent was evaporated under vacuum thereafter and the residue was further dried under high vacuum. The product was obtained as a hydrochloride salt at quantitative yield and was recrystallized from a 1:1 mixture of methanol and ether, filtered and dried.

¹H-NMR (CDCl₃): δ = 1.93 (quint, J = 7.14 Hz, 2H, CH₂CH₂NH₂), 2.23 (m, 2H, ArNCH₂CH₂), 2.61 (t, J = 7.14 Hz, 2H, CO₂CH₂), 3.01 (m, 2H, CH2NH2), 3.33 (m, 2H, ArNCH₂CH₂CH₂), 3.48-3.87 (m, 10H, five NCH₂), 4.10 (t, J = 6.4 Hz, 2H, NCH₂CH₂O), 4.48 (m, 2H, ArNCH₂), 7-7.31 (m, 7H, Ar) ppm.

¹³C-NMR (CDCl₃): δ = 22.34 (CH₂CH₂NH₂), 22.93 (ArNCH₂CH₂), 31.11 (CH₂CO₂), 39.56 (CH₂NH₂), 44.76 (ArNCH₂), 49.42 (two NCH₂), 49.61 (two NCH₂), 55.29 (ArCH₂CH₂CH₂), 56.08 (NCH₂CH₂O), 58.64 (NCH₂CH₂O), 116.69 (C₁₀), 117.20 (C₁), 123.49 (C₃), 124.19 (C₈), 125.44 (C₅), 126.42 (C₆), 128.20 (C₇), 128.56 (C₉), 128.80 (C₄),134.23 (C₂), 144.97 (C₁₂), 147.37 (C₁₁), 173.04 (CO₂) ppm.

MS (CI/CH₄): m/z (%) = 403.09 (MH+-C₄H₇NO, 100), 489.18 (MH+, 1.7).

The final product was found to be hygroscopic with water content of 1.05 % weight/weight as measured by the Karl Fischer (KF) titration analysis method.

The procedure described above was repeated on a similar scale except that after the 2 hours agitation at room temperature, heat was applied and the mixture was stirred at 40 °C for additional 2 hours. The HC1 salt was obtained as a tan sticky solid after filtration under a flow of nitrogen. The final product was found to have a water content of 12.5 % weight/weight as determined by KF analysis.

### REFERENCE EXAMPLE 3

### Synthesis of perphenazine N-Boc-4-amitiobutyrate (AN-197) - Route A

N-Boc-GABA (1.44 equivalent) and triethylamine (TEA, 1.44 equivalent) in a THF solution (5 volumes) were reacted with pivaloyl chloride (1.11 equivalent) to form the reactive mixed anhydride 4-(tert-butoxycarbonylamino)butanoic pivalic anhydride. This anhydride was then reacted with perphenazine (1.0 equivalent) for 16 hours at a temperature lower than 50 °C. The product was isolated at a yield greater than 90 %. HPLC analysis of the product showed that the product main peak is contaminated by an impurity (reflected as a "shoulder" in the HPLC chromatogram) of approximately 23 % by area. Further analysis showed that this impurity is 2-(4-(3-(2-chloro-10H-phenothiazin-10-yl)propyl)piperazin-1-yl)ethyl pivalate, the pivalate ester of perphenazine.

### REFERENCE EXAMPLE 4

### Synthesis of perphenazine N-Boc-4-aminobutyrate (AN-197)-Route B

N-Boc-GABA (1 equivalent) was reacted with perphenazine (1 equivalent) in the presence of dicyclohexylcarbodiimide (DCC, 1 equivalent) and 4-dimethylaminopyridine (DMAP, 0.3 equivalent) in anhydrous dichloromethane (DCM) for 4 hours, The mixture was filtered and the solvent was removed under vacuum. The residual oil was dissolved in acetonitrile, cooled to 0-5 °C for 1 hour and filtered. The acetonitrile was removed under vacuum and the residual oil was dissolved in ethyl acetate. The ethyl acetate solution was consecutively washed with citric acid solution, sodium bicarbonate and brine and concentrated under vacuum to afford AN-197 as an orange oil (97 % yield) which has a purity of 98.8 % as determined by HPLC.

### REFERENCE EXAMPLE 5

### Synthesis of perphenazine N-Boc-4-aminobutyrate (AN-197) - Route C

Perphenazine (10 grams, 24.8 mmol, 1.0 equivalent) and dichloromethane (60 ml, 6 volumes) were mixed in a clean dry N₂-flushed 500 ml round bottomed flask. 4-dimethylaminopyridine (DMAP, 0.91 gram, 7.4 mmol, 0.3 equivalent) and N-Boc-GABA (6.04 grams, 29.8 mmol, 1.2 equivalents) were added and the resulting cream slurry was cooled to 0-5 °C to attenuate the exothermic reaction. Dicyclohexylcarbodiimide (DCC, 6.44 grams, 31.2 mmol, 1.2 equivalents) was thereafter added in 1 gram portions and the slurry was warmed to room temperature and agitated overnight, while monitoring the depletion of the starting material by HPLC. Once the reaction was completed, the slurry was cooled to 0-5 °C for 2 hours, the formed byproduct 5,6-dihydrouracil (DHU) was filtered off and the filtrate was washed with cold (0-5 °C) dichloromethane (2 x 10 ml). The washed filtrate was concentrated to an oil under vacuum at 40 °C, and was redissolved thereafter in ethyl acetate (70 ml, 7 volumes) and cooled to 0-5 °C for 2 hours. The fine precipitating solids were filtered and washed with cold (0-5 °C) ethyl acetate (2 x 10 ml). The ethyl acetate solution was washed with 5 % citric acid solution (2 x 10 ml), 1 M sodium bicarbonate solution (2 x 10 ml) and brine (2 x 50 ml). The solution was concentrated under vacuum at 40 °C to afford the compound AN-197 as viscous orange oil (98 % yield), which has a purity of 99.47 % as determined by HPLC.

The above procedure was repeated three more times, using different amounts of the starting material, perphenazine and was found to be highly reproducible, by resulting in similar purity and yield levels of the product (AN-197). Specifically, two repeating experiments starting with 50 grams of perphenazine afforded AN-197 having a purity of 98.9 % and 99.07 %, at a yield of 98 % and 99 %, respectively. In a separate experiment, starting with 100 grams of perphenazine afforded AN-197 having a purity of 99.53 % and a yield of 81 %.

### REFERENCE EXAMPLE 6

### Stability studies of AN-197

The stability of AN-197 was determined by performing an HPLC analysis daily during a period of 24 days and determining the appearance of perphenazine and GABA, the degradation products of AN-197. A sample of AN-197, prepared according to the procedure described above in Example 4 (Route B), was dissolved in the mobile phase solution used in the HPLC analysis (2:1 acetonitrile:water, pH=8) and was kept at 20 °C. For comparison, a sample of neat AN-197 was kept under the same conditions and was similarly analyzed.

The results showed that when dissolved in the mobile phase solution, AN-197 exhibited a higher rate of degradation to perphenazine and GABA as compared to the degradation of the neat oil over the same time period. Thus, HPLC analysis of AN-197 solution in the HPLC mobile phase after 24 days showed an increase in the perphenazine content from 0.42 % to 7.98 %, whereby HPLC analysis of the neat oil showed only a slight breakdown to perphenazine and GABA over the 24 day period (an increase in the perphenazine content from 0.42 % to 0.79 %).

To evaluate the stability of AN-197 under conditions of a large scale production, characterized by longer procedure times, samples of AN-197, prepared according to the procedure described in Example 4 above (Route B) above, dissolved in dichloromethane, acetonitrile or ethyl acetate were heated to 40 °C overnight. Analysis of the samples following this time period showed that no degradation had occurred. It was therefore concluded that AN-197 is stable under such conditions.

To evaluate the stability of AN-197 under acidic conditions, a sample of AN-197, prepared according to the procedure described in Example 4 (Route B) above, was dissolved in ethyl acetate and stirred in a citric acid solution overnight. Analysis of the ethyl acetate solution before and after agitation in the presence of citric acid showed no hydrolysis of the product, demonstrating that citric acid may serve as the acid for choice in the work up procedure of the reaction product.

### REFERENCE EXAMPLE 7

### Stability studies of AN-168

To determine the stability of the HCl salt of the GABA conjugate (AN-168), prepared according to the procedure described in Example 2 above, three samples of the salt were studied by HPLC analyses. Thus, samples of AN-168 as dry salt were kept at 5 °C, 20 °C and 40 °C over a period of 24 day and were analyzed by HPLC for determining the appearance of degradation products. The results of these studies are presented in Table 2 below.

**Table 2**

| **Temp.** | **40 °C** | | **20 °C** | | **5 °C** | |
|---|---|---|---|---|---|---|
| **Time (hours)** | **AN-168** | **Perphenazine** | **AN-168** | **Perphenazine** | **AN-168** | **Perphenazine** |
| | **% area** | **% area** | **% area** | **% area** | **% area** | **% area** |
| **0** | 98.64 | 1.36 | 98.64 | 1.36 | 98.64 | 1.36 |
| **24** | 97.97 | 2.03 | 98.42 | 1.58 | 98.57 | 1.43 |
| **48** | 97.70 | 2.30 | 98.31 | 1.69 | 98.52 | 1.48 |
| **120** | 96.95 | 3.05 | 97.68 | 2.32 | 98.40 | 1.60 |
| **144** | 97.03 | 2.97 | 97.26 | 2.74 | 98.13 | 1.87 |
| **336** | 96.69 | 3.31 | 96.26 | 3.74 | 97.21 | 2.03 |
| **456** | 95.55 | 4.45 | 95.58 | 4.51 | 97.95 | 2.05 |
| **504** | 95.62 | 3.92 | 94.60 | 5.40 | 97.15 | 2.85 |
| **576** | 95.55 | 4.06 | 93.80 | 5.87 | 96.14 | 3.49 |

As can be seen in Table 2, AN-168 degraded into perphenazine and GABA and/or underwent trans-esterification to perphenazine and 2-pyrrolidinone over time, at each of the tested temperatures.

### EXAMPLE 8

### Preparation of addition salts of AN-197 with organic acids-general procedure

Exemplary addition salts of inorganic and organic acids of the chemical conjugates described herein were synthesized according to the general synthetic pathway exemplified in Scheme 1 below. The synthetic pathway of these salts generally includes (i) reacting perphenazine with N-protected-γ-aminobutyric acid so as to obtain an N-protected chemical conjugate thereof, as described hereinabove; and (ii) reacting the formed N-protected chemical conjugate with inorganic or organic acid, so as to afford the desired acid addition salt thereof.

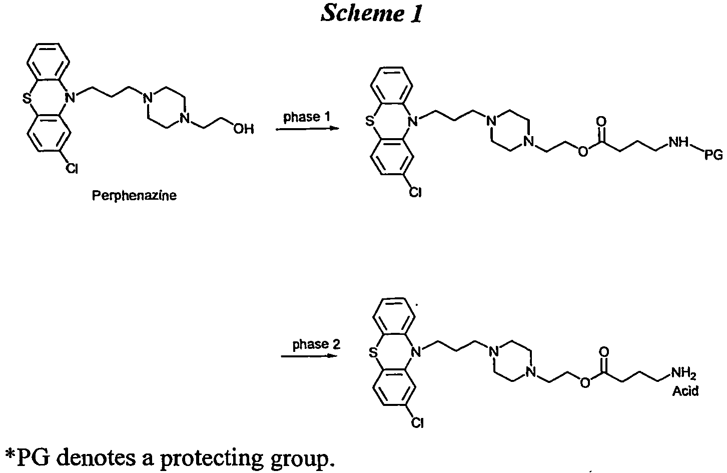

The addition salts of several organic acids and AN-197 were thus prepared according to the following general procedure: 3 grams of AN-197 were dissolved in an organic solvent, such as dichloromethane or ethyl acetate (10-20 ml, 2 volumes) under nitrogen atmosphere, while cooling the solution to 0-5 °C. A solution of trifluoroacetic acid (6-8 equivalents) was added to the AN-197 solution, and the mixture was allowed to warm up to room temperature or up to 35 °C, so as to remove the N-protecting group of the GABA residue. After 3-16 hours, when no more AN-197 was detected by HPLC, the reaction was concentrated under vacuum and the resulting oil was re-dissolved in 7 volumes of an organic solvent, and was quenched by means of addition of 1 M sodium bicarbonate (6-8 equivalents) at 0-5 °C, so as to bring the pH of the mixture to about 8. The quenching procedure was repeated 1-2 times, so as to afford the corresponding free base of AN-197.

A solution of an organic acid (1-3 equivalents) in an organic solvent, such as acetone or isopropyl alcohol, was added to the solution of the obtained free base (amine) of the conjugate and the mixture was there cooled to 0-5 °C, so as to precipitate the salt. The filtered solid salt was washed with the organic solvent, dried under vacuum and analyzed at room temperature by HPLC for traces of perphenazine and the free base of the conjugate.

Using the general procedure described above, various acid addition salts were prepared and analyzed so as to determine the synthetic efficiency and product stability thereof. Table 3 below presents the obtained results.

**Table 3**

| **No.** | **Acid** | **no. equiv** | **salt precipitation** | **acid: base ratio** | **molecular weight** |
|---|---|---|---|---|---|
| 1 * | succinic | 1.5/3.0 | no/no | | |
| 2 * | ascorbic | 1.0/3.0 | no/no | | |
| 3 | oxalic | 1.5/3.0 | yes/yes | 3:1 | 759.2 |
| 4 * | tartaric | 1.5/3.0 | no/no | | |
| 5 | maleic | 1.5/3.0 | yes/yes | 2:1 | 837.3 |
| 6 * | citric | 1.0/3.0 | no/no | | |
| 7 * | acetic | 1.5/3.0 | no/no | | |
| 8 * | phosphoric | 1.5/3.0 | no/no | | |
| 9 * | camphorsulfonic | 1.0/3.0 | no/no | | |
| 10 | methanesulfonic | 1.5/3.0 | yes/yes | 3:1 | 777.4 |

| | | | | | |
|---|---|---|---|---|---|
| *Reference example | | | | | |

### EXAMPLE 9

### Preparation of maleic acid addition salt of AN-197

The maleic acid addition salt of AN-197 was prepared according to the general procedure described above. The synthetic pathway is described in Scheme 2 below.

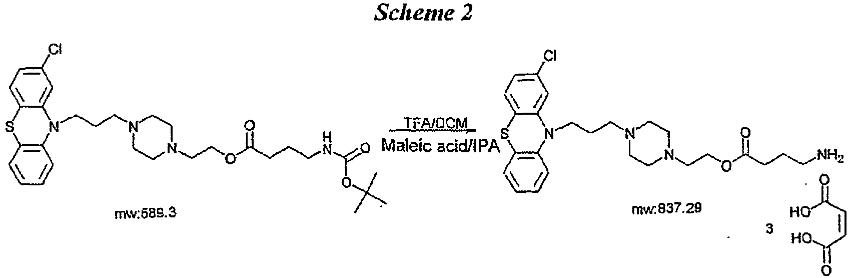

AN-197 (1.0 equivalent) and dichloromethane (DCM, 40 ml, 4 volumes) were mixed in a clean dry N₂-flushed 100 ml round bottomed flask and the mixture was stirred until all the starting material was dissolved. The solution was then cooled to 10 °C and trifluoroacetic acid (TFA, 7.7 equivalents) was added dropwise while maintaining the temperature below 20 °C. When the addition was completed, the resulting mixture was heated to 35 °C and maintained at this temperature for 16 hours. The obtained solution was then concentrated at 35 °C under vacuum, and the resulting oil was re-dissolved in DCM (7 volumes) and added to a stirred solution of 1 M sodium bicarbonate solution (7.7 equivalents) at 0-5 °C. The mixture was agitated at 0-5 °C for 15 minutes, the layers were thereafter separated and the lower organic layer was added to a stirred solution of 1 M sodium bicarbonate solution (7.7 equivalents) at 0-5 °C. The mixture was agitated at 0-5 °C for 15 minutes, the layers were thereafter separated and the organic layer was washed with water (5 volumes).

The layers were then separated again and a solution of maleic acid (3.0 equivalents) in isopropyl alcohol (IPA, 2 volumes) was added to the lower yellow organic layer, resulting in the precipitation of a yellow solid. The mixture was agitated at room temperature for 1 hour, then cooled to 0-5 °C for 1 hour, filtered and the filtrate was washed through with cold IPA (2 x 1 volume). The solid was then dried under vacuum at 40 °C to afford the tri-maleate salt as a yellow solid.

HPLC analysis of the solid product showed 98.31 % peak area for the salt, 0.82 % peak area for perphenazine and 0.01 % peak area for AN-197.

The preparation of the maleic acid addition salt presented hereinabove was successfully scaled up upon starting with 100 grams of AN-197. The maleate salt was isolated at a 98 % yield.

HPLC analysis of the product showed 99.31 % peak area for the salt, and 0.25 % peak area for perphenazine. The level of perphenazine at the large scale preparation was lower than previously seen at a smaller scale preparation, possibly due to lower levels of moisture ingress at the larger scale preparation.

The preparation of the maleic acid addition salt presented hereinabove was further successfully scaled up upon starting with 200 grams of AN-197, to afford the tri-maleate salt as a yellow solid with 97 % HPLC peak area for the salt.

### EXAMPLE 10

### Preparation of oxalic acid addition salt of AN-197

The oxalic acid addition salt of AN-197 was prepared according to the general procedure described above.

HPLC analysis of the product showed 98.05 % peak area for the salt and 0.75 % peak area for perphenazine.

### EXAMPLE 11

### Preparation of addition salts of AN-197 with organosulfonic acids - general procedure

Exemplary addition salts of organosulfonic acids of the chemical conjugates described herein were synthesized according to the general synthetic pathway exemplified in Scheme 3 below. The organosulfonic acid addition salts of AN-197 were prepared directly from AN-197, without separating the corresponding free base, as illustrated in Scheme 3 below. This one-step deprotection and *in-situ* salt formation approach further simplifies the salt formation process and minimizes the exposure of the free-base to moisture which may result in ester bond hydrolysis and hence in decomposition products. This process is made possible with organosulfonic acids since these acids are typically strong enough to remove the N-protecting group on the amino group of the chemical conjugates, avoiding the need to use TFA.

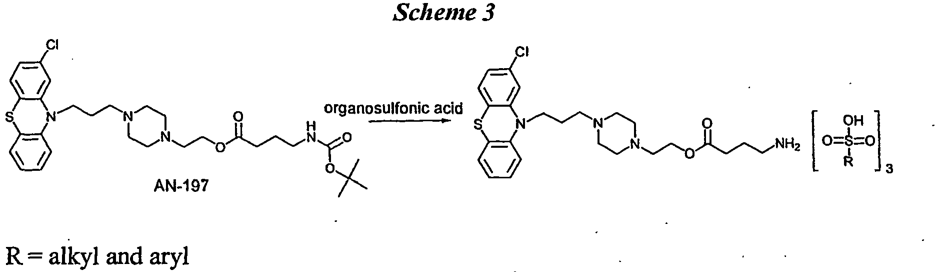

The addition salts of several organosulfonic acids and AN-197 were thus prepared according to the following general procedure: AN-197 (1.0 equivalent) and an organic solvent (40 ml, 4 volumes) were added to a clean dry N₂-flushed 100 ml round bottomed flask and the mixture was stirred until all the starting material was dissolved. The solution was heated to 40 °C and an organosulfonic acid (3.0 equivalents) was added. The resulting reaction mixture was heated at 40 °C for 5 hours, cooled to 0-5 °C and maintained at that temperature for 1 hour. The resulting slurry was filtered off under nitrogen, washed through with cold (0-5 °C) acetonitrile (2 x 1 volumes) and dried under vacuum at 40 °C to afford the tri-organosulfonate salt as a solid.

### EXAMPLE 12

### Preparation of methanesulfonic acid (mesylate) addition salt of AN-197

The methanesulfonic acid addition salt of AN-197 was prepared according to the general procedure described in Example 11 above. AN-197 (1.0 equivalent) and acetonitrile (MeCN, 40 ml, 4 volumes) were added to a clean dry N₂-flushed 100 ml round bottomed flask and the mixture was stirred until all the starting material was dissolved. The solution was heated to 40 °C and methanesulfonic acid (3.0 equivalents) was added. The resulting reaction mixture was heated at 40 °C for 5 hours, cooled to 0-5 °C and maintained at that temperature for 1 hour. The resulting slurry was filtered off under nitrogen, washed through with cold (0-5 °C) acetonitrile (2 x 1 volumes) and dried under vacuum at 40 °C to afford the tri-mesylate salt as a white solid.

HPLC analysis of the product showed 97.91 % peak area for the salt, 1.39 % peak area for perphenazine and 0.25 % peak area for AN-197.

The effect of the solvent was investigated by performing the reaction in dichloromethane, tetrahydrofuran, isopropyl acetate, methyl t-butyl ether or toluene as the reaction solvent. When isopropyl acetate, methyl tertiary butyl ether and toluene were used as solvents, the addition salt product did not precipitate and the product either remained in solution or oiled out of solution. Thus, acetonitrile and tetrahydrofurane were found to be the most suitable solvents, with acetonitrile being selected as an ideal solvent for further studies.

The above procedure was repeated three times with different amount of the starting material, AN-197, in order to assess its reproducibility. Reactions starting with 1 gram, 3 grams or 5 grams AN-197 were carried out and analyzed, resulting in 98.55 %, 98.93 % and 99.02 % peak area for the salt respectively, 0.29 %, 0.2 % and 0.39 % peak area for AN-197 respectively, and 1.08 %, 0.7 % and 0.31 % peak area for perphenazine respectively. These results are presented in Table 4 below.

**Table 4**

| **Starting material AN-197 (grams)** | **Sample tested** | **salt formed** | **HPLC peak area** | | |
|---|---|---|---|---|---|
| | | | **Salt (%)** | **AN-197 (%)** | **Perphenazine (%)** |
| 3 | Without drying | 3.0 | 98.93 | 0.2 | 0.7 |
| 5 | Without drying | 3.0 | 99.02 | 0.39 | 0.31 |
| 5 | With drying | 3.0 | 96.94 | 0.04 | 2.82 |
| 1 | Without drying | 3.0 | 98.55 | 0.29 | 1.08 |

The preparation of the methanesulfonic acid addition salt presented hereinabove was successfully scaled up. Starting with 100 grams of AN-197 afforded the methanesulfonic acid salt with 97.8 % HPLC peak area for the salt.

In an alternative route, the mesylate salt of AN-197 was prepared in acetone as follows:
AN-197 (1 gram, 1.7 mmol) and acetone (4 ml) were charged to a clean dry flask under nitrogen atmosphere and the mixture was warmed to 40 °C. After 5 minutes, AN-197 was completely dissolved and a clear solution was obtained. A solution of methanesulfonic acid (385 µl, 5.94 mmol, 3.5 equivalents), dissolved in acetone (2 ml) was then added and the resulting mixture was stirred to for 4 hours at 40 °C, under nitrogen atmosphere. The obtained product was precipitated from the solution during the reaction period, thus shifting the reaction towards product formation. The precipitate was then filtered under reduced pressure, washed with acetone and dried under reduced pressure, to afford 1.05 gram (83 % yield) of a mesylate addition salt having a 99.4 % purity, as determined by HPLC.

### EXAMPLE 13

### Preparation of maleic acid addition salt of AN-197 via deprotection by methanesulfonic acid

The process was designed so as to perform an initial deprotection of AN-197 with methanesulfonic acid, followed by neutralization and precipitation of the tri-maleate salt, while avoiding the use of TFA.

AN-197 (20 grams, 1.0 equivalent) and dichloromethane (DCM, 80 ml, 4 volumes) were mixed in a clean dry N₂-flushed 500 ml round bottomed flask and the mixture was stirred until all the starting material was dissolved.

Methanesulfonic acid (3.5 equivalents) was thereafter added dropwise while keeping the solution at 40 °C. After stirring for 4 hours the resulting mixture was cooled to room temperature and further cooled to 0-5 °C and maintained at that temperature for one hour. The reaction mixture in a form of a slurry was filtered under nitrogen atmosphere, washed with cold (0-5 °C) acetonitrile (4 x 1 volumes) and the resulting solid product was transferred to a 2 liter round bottomed flask. One volume of water (20 ml) was added thereafter to dissolve the salt, followed by the addition of dichloromethane (4 volumes). The mixture was washed twice with sodium bicarbonate (1 M, 2 x 7.7 equivalents) to generate the free base. The organic phase was further washed with 1 volume of water, and added slowly to 3 equivalents of maleic acid dissolved in isopropanol (70 ml). A yellowish solid precipitated and the slurry was cooled to 4 °C, filtered and dried at 40 °C under vacuum in an oven for 24 hours. The resulting tri-maleate salt was obtained at a purity higher than 96 % as determined by HPLC.

### REFERENCE EXAMPLE 14

### Stability studies of the free base (amine) of the chemical conjugate

The stability of neat free base of the chemical conjugate was determined by performing an HPLC analysis daily during a period of 24 days and determining the appearance of its degradation products perphenazine and GABA. Samples of the neat free base of the conjugate were collected after deprotection of AN-197 with TFA, as described in the preparation of the maleate and oxalate salts, using the general procedure described above in Example 9, were dissolved in the mobile phase solution which was used in the HPLC analysis (2:1 acetonitrile:water, pH 8) and were kept either at 20 °C or at 40 °C.

The results are presented in Table 6 below and indicate that the material is highly unstable at both temperatures, as demonstrated by the falling values of the relative peak area of the free base and the rising values of the relative peak area of perphenazine. During the first 24 hours approximately 5 % relative peak area degradation to perphenazine was observed in the sample kept at 20°C, while during the same time period more than 20 % of the free base degraded to perphenazine in the sample kept at 40 °C.

**Table 6**

| | | | | |
|---|---|---|---|---|
| **Temp.** | **40°C** | | **20 °C.** | |

| **Time (hours)** | **Free base** | **Perphenazine** | **Free base** | **Perphenazine** |
|---|---|---|---|---|
| | **% area** | **% area** | **% area** | **% area** |
| **0** | 98.50 | 1.50 | 98.50 | 1.50 |
| **24** | 78.74 | 21.26 | 95.05 | 4.95 |
| **168** | 20.09 | 79.91 | 74.71 | 25.29 |
| **336** | 6.12 | 93.88 | 61.63 | 38.37 |
| **504** | 5.52 | 94.48 | 53.35 | 46.65 |
| **576** | 3.93 | 96.07 | 47.23 | 52.77 |

### EXAMPLE 15

### Stability and hygroscopicity of the oxalate addition salts of the conjugate

A sample of the oxalate addition salt was kept in an open container at room temperature for a five-week period (about 840 hours) and analyzed thereafter. The hygroscopicity of the oxalate salt was determined by following the water content of the salt using the Karl Fischer (KF) titration analysis method and the chemical stability of the oxalate salt was determined by following the appearance of the degradation product perphenazine in HPLC analysis.

The initial water content of the oxalate salt was 1.36 weight percents, and at the end of the five-week period it increased to 1.90 weight percents.

The initial purity of the oxalate salt, as determined by HPLC, was 98.05 %, and at the end of the five-week period it decreased to 94.61 %.

The initial perphenazine content was 0.75 %, and at the end of the five-week period it increased to 1.71 %.

This study showed that the oxalate addition salt of A-197 is a more stable and less hygroscopic addition salt as compared to the HCl salt (AN-168).

### EXAMPLE P4 16

### Stability and hygroscopicity of the mesylate and maleate addition salts of the conjugate

Stability and hygroscopicity studies of the mesylate and maleate salts of the conjugate were carried out under various conditions. The hygroscopicity of the salts was determined by following the water content of the salt using the Karl Fischer (KF) titration analysis method. The chemical stability of the salts was determined by following the appearance of the degradation product perphenazine using HPLC analyses. The samples were kept at -20 °C under nitrogen, at room temperature in an open container, at room temperature in a sealed container and at 40 °C in a sealed container. The samples were tested at the end of the preparation procedure (t=0), 72 hours after the preparation time (t = 72), 1 week after the preparation time (t = 168) and two weeks after the preparation time (t = 336). The results obtained with the mesylate addition salt are presented in Table 7 below. The results obtained with the maleate addition salt are presented in Table 7 below.

**Table 7**

| **Elapsed time period before testing** | | **t=0 hours** | **t=72 hours** | **t = 168 hours** | **t=336 hours** |
|---|---|---|---|---|---|
| **Parameter tested** | **Retention time (minutes)** | **Peak area (%)** | **Peak area (%)** | **Peak area (%)** | **Peak area (%)** |
| **Sample condition** | **Sealed container kept at -20 °C under N₂** | | | | |
| **Water content %** | | 0.6 | 0.6 | 0.7 | 0.6 |
| **Salt** | 1.00 | 97.91 | 98.08 | 98.22 | 98.24 |
| **perphenazine** | 1.29 | 1.39 | 1.34 | 1.26 | 1.21 |
| **Unidentified impurity** | 1.45 | - | - | - | - |
| | | | | | |

| **Sample condition** | **Open container kept at room temperature** | | | | |
|---|---|---|---|---|---|
| **Water content** | | 0.6 | 1.2 | 4.0 | 4.8 |
| **Salt** | 1.00 | 97.91 | 90.30 | 81.40 | 61.97 |
| **perphenazine** | 1.29 | 1.39 | 8.53 | 17.68 | 35.74 |
| **Unidentified impurity** | 1.45 | - | - | - | 0.05 |
| | | | | | |

| **Sample condition** | **Sealed container kept at room temperature** | | | | |
|---|---|---|---|---|---|
| **Water content %** | | 0.6 | 0.8 | 1.3 | 1.5 |
| **Salt** | 1.00 | 97.91 | 95.84 | 94.98 | 91.67 |
| **Perphenazine** | 1.29 | 1.39 | 3.17 | 4.52 | 6.88 |
| **Unidentified impurity** | 1.45 | - | - | - | - |
| | | | | | |

| **Sample condition** | **Sealed container kept at 40 °C** | | | | |
|---|---|---|---|---|---|
| **Water content %** | | 0.6 | 0.8 | 1.2 | 0.9 |
| **Salt** | 1.00 | 97.91 | 91.83 | 91.91 | 87:17 |
| **perphenazine** | 1.29 | 1.39 | 7.33 | 7.56 | 11.63 |
| **Unidentified impurity** | 1.45 | - | - | - | - |

As can be seen in Table 7, the mesylate salt was mostly stable when kept in a sealed container at -20 °C and under nitrogen atmosphere. No significant changes in the chemical composition and water content were observed for this sample. The least stable sample was that kept in an open container at room temperature, possibly due to the rapid and significant water absorption of the salt which promoted degradation and de-esterification of the product. The mesylate salt which was held at room temperature open to the atmosphere has also become sticky in texture and changed color from pink to black.

**Table 8**

| **Elapsed time period before testing** | | **t=0 hours** | **t=72 hours** | **t =168 hours** | **t=336 hours** |
|---|---|---|---|---|---|
| **Parameter tested** | **Retention time (minutes)** | **Peak area (%)** | **Peak area (%)** | **Peak area (%)** | **Peak area (%)** |
| **Sample condition** | **Sealed container kept at -20 °C under N₂** | | | | |
| **Water content %** | | 0.3 | 0.3 | 0.3 | 0.5 |
| **Salt** | 1.00 | 96.54 | 96.79 | 96.85 | 96.69 |
| **perphenazine** | 1.29 | 1.44 | 1.38 | 1.39 | 1.39 |
| **Unidentified impurity** | 1.45 | 1.05 | 0.99 | 0.98 | 1.03 |
| | | | | | |

| **Sample condition** | **Open container kept at room temperature** | | | | |
|---|---|---|---|---|---|
| **Water content** | | 0.3 | 0.7 | 0.8 | 0.8 |
| **Salt** | 1.00 | 96.54 | 96.43 | 96.82 | 96.00 |
| **perphenazine** | 1.29 | 1.44 | 1.44 | 1.46 | 1.43 |
| **Unidentified impurity** | 1.45 | 1.05 | 0.98 | 0.94 | 0.93 |
| | | | | | |

| **Sample condition** | **Sealed container kept at room temperature** | | | | |
|---|---|---|---|---|---|
| **Water content %** | | 0.3 | 0.5 | 0.8 | 0.5 |
| **Salt** | 1.00 | 96.54 | 96.24 | 96.86 | 95.69 |
| **Perphenazine** | 1.29 | 1.44 | 1.43 | 1.40 | 1.47 |
| **Unidentified impurity** | 1.45 | 1.05 | 1.09 | 1.11 | 1.24 |
| | | | | | |

| **Sample condition** | **Sealed container kept at 40 °C** | | | | |
|---|---|---|---|---|---|
| **Water content %** | | 0.3 | 0.5 | 0.7 | 0.7 |
| **Salt** | 1.00 | 96.54 | 94.96 | 94.32 | 92.93 |
| **perphenazine** | 1.29 | 1.44 | 1.52 | 1.65 | 1.80 |
| **Unidentified impurity** | 1.45 | 1.05 | 2.16 | 2.46 | 2.79 |

As can be seen in Table 8, the maleate salt was stable in all sample forms. The least stable sample was that kept in an open container at room temperature, wherein slightly raised levels of water absorption of the salt were detected. Under these conditions, the maleate salt became slightly discolored, changing from yellow to light orange but stayed as a free flowing solid.

The sample of the maleate addition salt was further kept in an open container at room temperature for a five weeks period (about 840 hours) and analyzed thereafter. The water content of the sample reached 0.91 %, the purity decreased to 94.98 %, and the perphenazine increased to 1.46 %.

This study showed that the maleate addition salt is a more stable and less hygroscopic addition salt as compared to the HCl and mesylate salt.

### EXAMPLE 17

### Solubility studies of mesylate and maleate addition salts of the conjugate

1.0 gram of each of the mesylate and maleate addition salts prepared as described herein was agitated in 10 ml of 1 % weight/volume lactic acid solution for 1 hour at room temperature. Any solids remaining thereafter were filtered off and the filtrate was dried under vacuum until no further change in weight was detected. The calculated solubility obtained for the maleate salt was 48 mg/ml. The calculated solubility obtained for the mesylate salt was more than 100 mg/ml.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

## Claims

1. A chemical conjugate comprising a first chemical moiety covalently linked to a second chemical moiety, wherein said first chemical moiety is a perphenazine residue as a residue of an anti-psychotic drug and further wherein said second chemical moiety is a γ-aminobutyric acid (GABA) residue as a residue of a GABA agonist which contains an amino group, whereas said amino group is in the form of an acid addition salt thereof,
wherein said acid addition salt is selected from the group consisting of a maleic acid addition salt, a methanesulfonic acid addition salt, and an oxalic acid addition salt.

2. The chemical conjugate of claim 1, being chemically stable under storage at a temperature that ranges from -50 °C and 50 °C for a time period of at least 7 days.

3. The chemical conjugate of claim 2, wherein a change in the purity of the chemical conjugate upon said storage is less than 4 percents of the initial purity of the conjugate.

4. The chemical conjugate of claim 1, being characterized as non-hygroscopic.

5. The chemical conjugate of claim 4, wherein a change in a water content of the chemical conjugate upon storage at a temperature that ranges from -50°C. and 50 °C is less than 0.4 weight percent of the total weight of the conjugate.

6. The chemical conjugate of claim 5, wherein said storage is for a time period of at least 14 days.

7. A pharmaceutical composition comprising, as an active ingredient, the chemical conjugate of claim 1 and a pharmaceutically acceptable carrier.

8. The pharmaceutical composition of claim 7, being packaged in a packaging material and identified in print, on or in said packaging material, for use in the treatment of a psychotic disease or disorder.

9. The chemical conjugate of claim 1 for use in the treatment of a psychotic disorder or disease.

10. The chemical conjugate of claim 9, wherein said psychotic disorder or disease is Schizophrenia.

11. The chemical conjugate, or pharmaceutical composition of any of claims 1-10, wherein said second chemical moiety is covalently linked to said first chemical moiety via an ester bond selected from the group consisting of a carboxylic ester bond, an alkyloxy carboxylic ester bond, an amide bond and a thioester bond.

12. A process of preparing the chemical conjugate of any of claims 1-6 or 9 to 11, the process comprising:
providing a N-protected chemical conjugate including said first chemical moiety being covalently linked to said second chemical moiety, whereas said amino group is protected by an N-protecting group;
removing said N-protecting group to thereby provide a free base form of said chemical conjugate; and
contacting said free base form of said chemical conjugate with a first acid selected from the group consisting of maleic acid, methanesulfonic acid, and oxalic acid, thereby providing the chemical conjugate.

13. The process of claim 12, wherein providing said N-protected chemical conjugate comprises:
reacting said anti-psychotic drug with an N-protected organic acid.

14. The process of claim 13, further comprising, prior to said reacting:
reacting said N-protected organic acid with an acyl halide to thereby obtain a mixed anhydride derivative of said N-protected organic acid.

15. The process of claim 13, wherein said reacting is performed in the presence of a solvent, an organic base and a dehydrating agent.

16. The process of claim 15, further comprising, prior to said reacting:
mixing said anti-psychotic drug, said organic base and said N-protected organic acid at a temperature that ranges from about 0 °C to about 5 °C, to thereby obtain a slurry;
adding said dehydrating agent to said slurry; and
allowing said slurry to warm to room temperature.

17. The process of claim 12, wherein said removing said protecting group and said contacting are performed successively.

18. The process of claim 17, wherein said removing said protecting group is effected by contacting the chemical conjugate with a second acid.

19. The process of claim 24, wherein said second acid is selected from the group consisting of trifluoroacetic acid and methanesulfonic acid.

20. The process of claim 12, wherein said removing and said contacting are performed concomitantly without isolating said free base form of said conjugate.

21. The process of claim 20, wherein said first acid is methanesulfonic acid.

22. The process of claim 21, the process further comprising, subsequent to said contacting:
adding an anti-solvent to thereby precipitate the chemical conjugate.

23. The process of claim 21, wherein said removing and said contacting are performed in the presence of a a solvent, said solvent being selected such that said first acid and said N-protected chemical conjugate are dissolvable therein and the chemical conjugate is precipitated therefrom.

24. The process of any of claims 12-23, wherein the chemical conjugate has a purity that equals to or is greater than 97 percents.

25. A process of preparing the chemical conjugate of any of claims 1 to 6 or 9 to 11, the process comprising:
reacting said organic acid with an N-protecting group, to thereby obtain an N-protected organic acid;
reacting said N-protected organic acid with said anti-psychotic drug; and
removing said N-protecting group, thereby obtaining the conjugate.

26. The process of claim 25, further comprising, prior to reacting said N-protected organic acid with said anti-psychotic drug:
reacting said N-protected organic acid with an acyl halide, to thereby obtain a mixed anhydride derivative of said N-protected organic acid.

27. The process of claim 25, wherein reacting said N-protected organic acid with said anti-psychotic drug is performed in the presence of a solvent, an organic base and a dehydrating agent.

28. The process of claim 27, further comprising, prior to said reacting:
mixing said anti-psychotic drug, said organic base and said N-protected organic acid at a temperature that ranges from about 0 °C to about 5 °C, to thereby obtain a slurry;
adding said dehydrating agent to said slurry; and
allowing said slurry to warm to room temperature.

29. The process of any of claims 13-28, wherein said second chemical moiety is covalently linked to said first chemical moiety via an ester bond selected from the group consisting of a carboxylic ester bond, an alkyloxy carboxylic ester bond, an amide bond and a thioester bond.

30. The chemical conjugate of claim 1, comprising a perphenazine residue covalently linked to a γ-aminobutyric acid (GABA) residue, whereas an amino group of said γ-aminobutyric acid (GABA) residue is in the form of a methanesulfonic acid (mesylate) addition salt thereof.

## Patentansprüche

1. Ein chemisches Konjugat, welches eine erste chemische Einheit umfasst, welche kovalent an eine zweite chemische Einheit gebunden ist, wobei die erste chemische Einheit ein Perphenazinrest als ein Rest eines anti-psychotischen Medikaments ist, und wobei ferner die zweite chemische Einheit ein γ-Aminobuttersäurerest (GABA) als ein Rest eines GABA-Agonisten ist, welcher einen Aminrest enthält, wobei der Aminorest in Form eines Säureadditionssalzes davon vorliegt,
wobei das Säureadditionssalz ausgewählt ist aus der Gruppe, bestehend aus einem Maleinsäureadditionssalz, einem Methansulfonsäureadditionssalz und einem Oxalsäureadditionssalz.

2. Das chemische Konjugat nach Anspruch 1, welches bei der Lagerung bei einer Temperatur, die von -50°C bis 50°C reicht, über einen Zeitraum von mindestens 7 Tagen chemisch stabil ist.

3. Das chemische Konjugat nach Anspruch 2, wobei eine Änderung der Reinheit des chemischen Konjugats während der Lagerung weniger als 4 Prozent der ursprünglichen Reinheit des Konjugats beträgt.

4. Das chemische Konjugat nach Anspruch 1, **dadurch gekennzeichnet, dass** es nichthygroskopisch ist.

5. Das chemische Konjugat nach Anspruch 4, wobei eine Änderung des Wassergehalts des chemischen Konjugats während der Lagerung bei einer Temperatur, welche von -50 bis 50°C reicht, weniger als 0,4 Gewichtsprozent des Gesamtgewichts des Konjugats beträgt.

6. Das chemische Konjugat nach Anspruch 5, wobei die Lagerung über einen Zeitraum von mindestens 14 Tagen andauert.

7. Eine pharmazeutische Zusammensetzung, welche als wirksamen Bestandteil das chemische Konjugat nach Anspruch 1 und einen pharmazeutisch verträglichen Träger umfasst.

8. Die pharmazeutische Zusammensetzung nach Anspruch 7, welche in einem Verpackungsmaterial verpackt ist und in gedruckter Form auf oder in dem Verpackungsmaterial zum Gebrauch bei der Behandlung einer psychotischen Krankheit oder Störung gekennzeichnet ist.

9. Das chemische Konjugat nach Anspruch 1 zur Verwendung bei der Behandlung einer psychotischen Störung oder Krankheit.

10. Das chemische Konjugat nach Anspruch 9, wobei die psychotische Störung oder Krankheit Schizophrenie ist.

11. Das chemische Konjugat oder die pharmazeutische Zusammensetzung nach einem der Ansprüche 1-10, wobei die zweite chemische Einheit über eine Esterbindung, ausgewählt aus der Gruppe bestehend aus einer Carbonsäureesterbindung, einer Alkyloxycarbonsäureesterbindung, einer Amidbindung und einer Thioesterbindung, kovalent an die erste chemische Einheit gebunden ist.

12. Ein Verfahren zur Herstellung des chemischen Konjugats nach einem der Ansprüche 1-6 oder 9 bis 11, wobei das Verfahren umfasst:
Bereitstellen eines N-geschützten chemischen Konjugats, welches die erste chemische Einheit, die kovalent an die zweite chemische Einheit gebunden ist, enthält, wobei der Aminrest durch eine N-Schutzgruppe geschützt ist;
Entfernen der N-Schutzgruppe, um eine freie Basenform des chemischen Konjugats bereit zu stellen; und
in Kontakt bringen der freien Basenform des chemischen Konjugats mit einer ersten Säure, ausgewählt aus der Gruppe bestehend aus Maleinsäure, Methansulfonsäure und Oxalsäure, um das chemische Konjugat bereit zu stellen.

13. Das Verfahren nach Anspruch 12, wobei das Bereitstellen des N-geschützten chemischen Konjugats umfasst:
Umsetzen des anti-psychotischen Medikaments mit einer N-geschützten organischen Säure.

14. Das Verfahren nach Anspruch 13, weiter umfassend vor dem Umsetzen:
Umsetzen der N-geschützten organischen Säure mit einem Acylhalogenid, um ein gemischtes Anhydridderivat der N-geschützten organischen Säure zu erhalten.

15. Das Verfahren nach Anspruch 13, wobei das Umsetzen in der Gegenwart eines Lösungsmittels, einer organischen Base und eines Entwässerungsmittels durchgeführt wird.

16. Das Verfahren nach Anspruch 15, welches vor dem Umsetzen weiter umfasst:
Mischen des anti-psychotischen Medikaments, der organischen Base und der N-geschützten organischen Säure bei einer Temperatur, welche von etwa 0°C bis etwa 5°C reicht, um eine Aufschlämmung zu erhalten;
Zugeben des Entwässerungsreagenzes zu der Aufschlämmung; und
Zulassen des Erwärmens der Aufschlämmung auf Raumtemperatur.

17. Das Verfahren nach Anspruch 12, wobei das Entfernen der Schutzgruppe und das in Kontakt bringen nacheinander durchgeführt werden.

18. Das Verfahren nach Anspruch 17, wobei das Entfernen der Schutzgruppe durch in Kontakt bringen des chemischen Konjugats mit einer zweiten Säure erfolgt.

19. Das Verfahren nach Anspruch 18, wobei die zweite Säure aus der Gruppe bestehend aus Trifluoressigsäure und Methansulfonsäure ausgewählt ist.

20. Das Verfahren nach Anspruch 12, wobei das Entfernen und das in Kontakt bringen gleichzeitig ohne Isolieren der freien Basenform des Konjugats durchgeführt werden.

21. Das Verfahren nach Anspruch 20, wobei die erste Säure Methansulfonsäure ist.

22. Das Verfahren nach Anspruch 21, wobei das Verfahren im Anschluss an das in Kontakt bringen weiter umfasst:
Zugeben eines Nichtlösungsmittels, um das chemische Konjugat auszufällen.

23. Das Verfahren nach Anspruch 21, wobei das Entfernen und das in Kontakt bringen in der Gegenwart eines Lösungsmittels durchgeführt werden, wobei das Lösungsmittel so ausgewählt ist, das die erste Säure und das N-geschützte Konjugat darin löslich sind und das chemische Konjugat daraus ausgefällt wird.

24. Das Verfahren nach einem der Ansprüche 12-23, wobei das chemische Konjugat eine Reinheit hat, die gleich oder größer 97 Prozent ist.

25. Ein Verfahren zur Herstellung des chemischen Konjugats nach einem der Ansprüche 1 bis 6 oder 9 bis 11, wobei das Verfahren umfasst:
Umsetzen der organischen Säure mit einer N-Schutzgruppe, um eine N-geschützte organische Säure zu erhalten;
Umsetzen der N-geschützten organischen Säure mit dem anti-psychotischen Medikament; und
Entfernen der N-Schutzgruppe, um das Konjugat zu erhalten.

26. Das Verfahren nach Anspruch 25, welches vor dem Umsetzen der N-geschützten organischen Säure mit dem anti-psychotischen Medikament weiter umfasst:
Umsetzen der N-geschützten organischen Säure mit einem Acylhalogenid, um ein gemischtes Anhydridderivat der N-geschützten organischen Säure zu erhalten.

27. Das Verfahren nach Anspruch 25, wobei Umsetzen der N-geschützten organischen Säure mit dem anti-psychotischen Medikament in Gegenwart eines Lösungsmittels, einer organischen Base und eines Entwässerungsmittels durchgeführt wird.

28. Das Verfahren nach Anspruch 27, welches vor dem Umsetzen weiter umfasst:
Mischen des anti-psychotischen Medikaments, der organischen Base und der N-geschützten organischen Säure bei einer Temperatur, welche von etwa 0°C bis etwa 5°C reicht, um eine Aufschlämmung zu erhalten;
Zugeben des Entwässerungsreagenzes zu der Aufschlämmung; und
Zulassen des Erwärmens der Aufschlämmung auf Raumtemperatur.

29. Das Verfahren nach einem der Ansprüche 13-28, wobei die zweite chemische Einheit über eine Esterbindung, ausgewählt aus der Einheit bestehend aus einer Carbonsäureesterbindung, einer Alkyloxycarbonsäureesterbindung, einer Amidbindung und einer Thioesterbindung, kovalent an die erste chemische Einheit gebunden ist.

30. Das chemische Konjugat nach Anspruch 1, welches einen Perphenazinrest, kovalent gebunden an einen γ-Aminobuttersäurerest (GABA), umfasst, wobei ein Aminrest des γ-Aminobuttersäurerestes (GABA) in Form eines Methansulfonsäureadditionssalzes davon (Mesylat) vorliegt.

## Revendications

1. Conjugué chimique comprenant un premier fragment chimique lié de manière covalente à un second fragment chimique, dans lequel ledit premier fragment chimique est un résidu perphénazine en tant que résidu d'un médicament antipsychotique et en outre dans lequel ledit second fragment chimique est un résidu acide γ-aminobutyrique (GABA) en tant que résidu d'un agoniste de GABA qui contient un groupe amino, tandis que ledit groupe amino est sous la forme d'un sel d'addition d'acide de celui-ci,
dans lequel ledit sel d'addition d'acide est choisi dans le groupe constitué par un sel d'addition d'acide maléique, un sel d'addition d'acide méthanesulfonique, et un sel d'addition d'acide oxalique.

2. Conjugué chimique selon la revendication 1, chimiquement stable en stockage à une température qui va de -50 à 50 °C pendant une période de temps d'au moins 7 jours.

3. Conjugué chimique selon la revendication 2, dans lequel une variation de pureté du conjugué chimique lors dudit stockage est inférieure à 4 % de la pureté initiale du conjugué.

4. Conjugué chimique selon la revendication 1, caractérisé comme étant non hygroscopique.

5. Conjugué chimique selon la revendication 4, dans lequel une variation de la teneur en eau du conjugué chimique lors d'un stockage à une température qui va de -50 à 50 °C est inférieure à 0,4 % en poids du poids total du conjugué.

6. Conjugué chimique selon la revendication 5, dans lequel ledit stockage dure pendant une période d'au moins 14 jours.

7. Composition pharmaceutique comprenant, à titre de principe actif, le conjugué chimique selon la revendication 1 et un véhicule pharmaceutiquement acceptable.

8. Composition pharmaceutique selon la revendication 7, emballée dans un matériau d'emballage et identifiée par des caractères imprimés, sur ou dans ledit matériau d'emballage, pour une utilisation dans le traitement d'une maladie ou d'un trouble psychotique.

9. Conjugué chimique selon la revendication 1 pour une utilisation dans le traitement d'un trouble ou d'une maladie psychotique.

10. Conjugué chimique selon la revendication 9, dans lequel ledit trouble ou ladite maladie psychotique est la schizophrénie.

11. Conjugué chimique ou composition pharmaceutique selon l'une quelconque des revendications 1 à 10, dans lequel ledit second fragment chimique est lié par covalence audit premier fragment chimique par l'intermédiaire d'une liaison ester choisie dans le groupe constitué par une liaison ester carboxylique, une liaison ester alkyloxycarboxylique, une liaison amide et une liaison thioester.

12. Procédé de préparation du conjugué chimique selon l'une quelconque des revendications 1 à 6 ou 9 à 11, le procédé comprenant :
la préparation d'un conjugué chimique N-protégé comprenant ledit premier fragment chimique lié de manière covalente audit second fragment chimique, tandis que ledit groupe amino est protégé par un groupe N-protecteur ;
l'élimination dudit groupe N-protecteur pour obtenir ainsi une forme de base libre dudit conjugué chimique ; et
la mise en contact de ladite forme de base libre dudit conjugué chimique avec un premier acide choisi dans le groupe constitué par l'acide maléique, l'acide méthanesulfonique, et l'acide oxalique, pour obtenir ainsi le conjugué chimique.

13. Procédé selon la revendication 12, dans lequel la préparation dudit conjugué chimique N-protégé comprend :
la réaction dudit médicament antipsychotique avec l'acide organique N-protégé.

14. Procédé selon la revendication 13 comprenant en outre, avant ladite réaction :
la réaction dudit acide organique N-protégé avec un halogénure d'acyle pour obtenir ainsi un dérivé d'anhydride mixte dudit acide organique N-protégé.

15. Procédé selon la revendication 13, dans lequel ladite réaction est mise en oeuvre en présence d'un solvant, d'une base organique et d'un agent de déshydratation.

16. Procédé selon la revendication 15, comprenant en outre, avant ladite réaction :
le mélange dudit médicament antipsychotique, de ladite base organique et dudit acide organique N-protégé à une température qui va d'environ 0 à environ 5 °C, pour obtenir ainsi un fluide chargé ;
l'ajout dudit agent de déshydratation audit fluide chargé ; et
le réchauffement tel quel dudit fluide chargé jusqu'à la température ambiante.

17. Procédé selon la revendication 12, dans lequel ladite élimination dudit groupe protecteur et ladite mise en contact sont mises en oeuvre successivement.

18. Procédé selon la revendication 17, dans lequel ladite élimination dudit groupe protecteur est mise en oeuvre par mise en contact du conjugué chimique avec un second acide.

19. Procédé selon la revendication 18, dans lequel ledit second acide est choisi dans le groupe constitué par l'acide trifluoracétique et l'acide méthanesulfonique.

20. Procédé selon la revendication 12, dans lequel ladite élimination et ladite mise en contact sont mises en oeuvre concomitamment sans isoler ladite forme de base libre dudit conjugué.

21. Procédé selon la revendication 20, dans lequel ledit premier acide est l'acide méthanesulfonique.

22. Procédé selon la revendication 21, le procédé comprenant en outre, après ladite mise en contact :
l'ajout d'un anti-solvant pour précipiter ainsi le conjugué chimique.

23. Procédé selon la revendication 21, dans lequel ladite élimination et ladite mise en contact sont mises en oeuvre en présence d'un solvant, ledit solvant étant choisi pour que ledit premier acide et ledit conjugué chimique N-protégé puissent être dissous dans celui-ci et que le conjugué chimique soit précipité à partir de celui-ci.

24. Procédé selon l'une quelconque des revendications 12 à 23, dans lequel le conjugué chimique a une pureté qui est égale ou supérieure à 97 %.

25. Procédé de préparation du conjugué chimique selon l'une quelconque des revendications 1 à 6 ou 9 à 11, le procédé comprenant :
la réaction dudit acide organique avec un groupe N-protecteur, pour obtenir ainsi un acide organique N-protégé ;
la réaction dudit acide organique N-protégé avec ledit médicament antipsychotique ; et
l'élimination du groupe N-protecteur, pour obtenir ainsi le conjugué.

26. Procédé selon la revendication 25, comprenant en outre, avant la réaction dudit acide organique N-protégé avec ledit médicament antipsychotique :
la réaction dudit acide organique N-protégé avec un halogénure d'acyle, pour obtenir ainsi un dérivé d'anhydride mixte dudit acide organique N-protégé.

27. Procédé selon la revendication 25, dans lequel la réaction dudit acide organique N-protégé avec ledit médicament antipsychotique est mise en oeuvre en présence d'un solvant, d'une base organique et d'un agent de déshydratation.

28. Procédé selon la revendication 27, comprenant en outre, avant ladite réaction :
le mélange dudit médicament antipsychotique, de ladite base organique et dudit acide organique N-protégé à une température qui va d'environ 0 à environ 5 °C, pour obtenir ainsi un fluide chargé ;
l'ajout dudit agent de déshydratation audit fluide chargé ; et
le réchauffement tel quel dudit fluide chargé jusqu'à la température ambiante.

29. Procédé selon l'une quelconque des revendications 13 à 28, dans lequel ledit second fragment chimique est lié de manière covalente audit premier fragment chimique par l'intermédiaire d'une liaison ester choisie dans le groupe constitué par une liaison ester carboxylique, une liaison ester alkyloxycarboxylique, une liaison amide et une liaison thioester.

30. Conjugué chimique selon la revendication 1, comprenant un résidu perphénazine lié de manière covalente à un résidu acide γ-aminobutyrique (GABA), tandis qu'un groupe amino dudit résidu acide γ-aminobutyrique (GABA) est sous la forme d'un sel d'addition d'acide méthanesulfonique (mésylate) de celui-ci.
